# EUROPÄISCHE PATENTSCHRIFT

(11) **EP 2 176 170 B1**
(45) Veröffentlichungstag und Bekanntmachung des Hinweises auf die Patenterteilung: **15.03.2017**
(21) Anmeldenummer: 09700283.6
(22) Anmeldetag: 09.01.2009
(51) Int. Cl.: C01G 49/02, C01G 49/06, C01G 49/08, C09C 1/24, A61K 41/00, B01J 19/10, A61K 33/26, A61K 45/06, A61K 9/14, B82Y 30/00

(54) **MAGNETISCHE TRANSDUCER**
MAGNETIC TRANSDUCER
TRANSDUCTEURS MAGNÉTIQUES

(30) Priorität: 09.01.2008 DE 102008003615
(43) Veröffentlichungstag der Anmeldung: 21.04.2010
(73) Patentinhaber: MagForce AG, 12489 Berlin (DE)
(72) Erfinder: WALDÖFNER, Norbert, 47167 Duisburg (DE); STIEF, Kerstin, 10247 Berlin (DE)
(74) Vertreter: Bösl, Raphael Konrad
(86) Internationale Anmeldenummer: PCT/DE2009/000038
(87) Internationale Veröffentlichungsnummer: WO 2009/086824

(56) Entgegenhaltungen:
- DE-A1- 2 543 962
- DE-A1- 19 614 136
- SHOUHENG SUN, HAO ZENG, DAVID B. ROBINSON, SIMONE RAOUX, PHILIP M. RICE, SHAN X. WANG, AND GUANXIONG LI: "Monodisperse MFe2O4 (M = Fe, Co, Mn) Nanoparticles" J. AM. CHEM. SOC., Bd. 126, Nr. 1, 2004, Seiten 273-279, XP002542496
- A.K. GUPTA, M. GUPTA: "Synthesis and surface engineering of iron oxide nanoparticles for biomedical applications" BIOMATERIALS, Bd. 26, Nr. 18, 2005, Seiten 3995-4021, XP002542497
- A. ZABLOTSKAYA, I. SEGAL, M. MAIOROV, D. ZABLOTSKY, A. MISHNEV, E. LUKEVICS, I. SHESTAKOVA AND I. DOMRACHEV: "Synthesis and characterization of nanoparticles with an iron oxide magnetic core and a biologically active trialkylsilylated aliphatic alkanolamine shell" JOURNAL OF MAGNETISM AND MAGNETIC MATERIALS, Bd. 311, Nr. 1, 2007, Seiten 135-139, XP002542498

## Beschreibung

Die vorliegende Erfindung betrifft die Herstellung biokompatibler magnetischer Nanopartikel, die, wenn sie einem magnetischen Wechselfeld ausgesetzt werden, ein hohes Maß an Wärme erzeugen. Die erzeugte Wärme kann unter anderem für therapeutische Zwecke genutzt werden, insbesondere für die Bekämpfung von Krebs.

Magnetische Nanopartikel können auf verschiedene Art und Weise die Energie eines magnetischen Wechselfeldes in Wärme umwandeln. Neben dem Aufheizen durch so genannte Hysterese-Verluste können Nanopartikel durch Relaxation (Neel bzw. Brown'sche Relaxation) Wärme erzeugen. Die Menge der erzeugten Wärmeenergie ist abhängig von der Magnetfeldstärke (Amplitude) und der Frequenz des Wechselfeldes. Die Effizienz der Wärmeerzeugung kann, bei definierter Stärke und Frequenz des Magnetfeldes, durch den so genannten SAR- bzw. SLP-Wert (SAR = Specific Absorption Rate, SPL = Specific Power Loss) beziffert werden. SAR-Werte einer Substanz werden auf die zur Messung eingesetzte Masse (in Gramm) normiert und in der Einheit [W/g] angegeben. Der SAR-Wert einer magnetischen Substanz hangt jedoch noch von anderen Faktoren, wie z.B. der Partikelgröße, der Partikelform, der Anisotropie und dem Metallgehalt der Substanz ab. Die SAR wird vorzugsweise gemäß einer von Jordan et al. entwickelten Methode [International Journal of Hyperthermia, 1993, Vol. 9, No. 1, 51-68] bei einer Frequenz von 100 kHz und einer Feldstärke von bis zu 18 KA/m bestimmt. Die Angabe des SAR-Weries erfolgt hier nach einer Normierung auf den Eisengehalt der Substanz in mW/mg Fe.

### Stand der Technik

Biokompatible magnetische Nanopartikel werden häufig durch ein so genanntes Fällungsverfahren hergestellt. Hierfür gibt es in der Literatur zahlreiche Beispiele [z.B. DE 196 14 136 A1]. Da diese Partikel in wässriger Lösung hergestellt werden, können sie problemlos funktionalisiert werden und besitzen meistens eine gute Biokompatibilität. Die auf diese Art und Weise hergestellten Partikel weisen allerdings verhältnismäßig geringe SAR-Werte auf und können somit den erfindungsgemäßen Anspruch dieses Patentes nicht erfüllen.

Magnetische Nanopartikel können auch über so genannte magnetotaktische Bakterien hergestellt werden [WO 98/40049]. Die auf diese Weise hergestellten Nanopartikel besitzen eine höhere SAR. Jedoch ist der Herstellungsprozess sehr komplex und kostspielig. Darüber hinaus sedimentieren die Partikel relativ schnell, was die Anwendungsmöglichkeiten stark einschränkt.

Seit längerem ist bekannt, dass durch thermische Zersetzung von Metallkomplexen in organischen Lösungsmitteln Kolloide bzw. Nanopartikel entstehen [z.B. Smith et al., J. Phys. Chem. 1980, 84, 1621-1629]. Monodisperse Partikel in unterschiedlichen Größen können nach den von Peng et al. [US 2006/0211152 A1] und Hyeon et al. [WO 2006/057533 A1] veröffentlichen Verfahren hergestellt werden. Jedoch sind die nach diesen Verfahren hergestellten Partikel nur in organischen Lösungsmitteln dispergierbar und somit nicht biokompatibel. Darüber hinaus sind die SAR-Werte der nach diesen Verfahren hergestellten Partikel gering. Die Dispergierung von solchen (hydrophoben) Partikeln in Wasser kann durch eine Modifizierung der Hülle zwar prinzipiell erreicht werden [z.B. Wang et al., Nano Lett., 2003, 3(11), 1555-1559 oder De Palma et al., Chem. Mater, 2007, 19, 1821-1831]. Diese Methoden basieren auf einem direkten Austausch von hydrophoben Liganden gegen hydrophile Liganden. Diese Beschichtungsmethoden liefern nur eine dünne (Monolayer) Beschichtung, die nicht den erfindungsgemäßen Anspruch einer stabilen biokompatiblen Beschichtung erfüllt. Die kolloidale Stabilität der Partikel ist zudem eingeschränkt, so dass die erfindungsgemäßen Partikel mit dieser Methode nicht beschichtet werden können. Zudem können nur stark verdünnte Partikel-Dispersionen beschichtet werden. Demnach existiert keine technische befriedigende Lösung für die Dispergierung der erfindungsgemäßen Partikel im industriellen Maßstab. Zudem besitzen die zur Dispergierung eingesetzten Substanzen bzw. Lösungsmittel in der Regel eine zu hohe Toxizität, was die Biokompatibilität einschränkt.

Biokompatible Eisenoxid-Nanopartikel können auch gemäß DE 196 14 136 A1 durch eine Beschichtung mit Silanen erhalten werden, jedoch ist diese Methode nur anwendbar, wenn die Partikel bereits in Wasser dispergiert sind, wohingegen hydrophobe Partikel nicht ohne weiteres mit Silanen oder Silika beschichtet werden können.

Sun et al. (J. AM. CHEM. SOC. 2004, 126, 273-279) beschreiben ein Verfahren zur Herstellung magnetischer Eisenoxidpartikel, wobei das Verfahren nicht die Verfahrensschritte E1) bis K1) der vorliegenden Erfindung umfasst. Die Offenlegungsschrift DE 196 14 136 A1 beschreibt ein Verfahren zur Herstellung agglomeratfreier nanoskaliger Eisenoxidteilchen mit hydrolysebeständigem Überzug. Die Offenlegungsschrift 25 43 962 betrifft Eisenoxid-Magnetpigmente für die Herstellung von Magnetschichten, wobei insbesondere ein Verfahren zur Verbesserung der Dispergierbarkeit und Erhöhung der Abriebfestigkeit von feinteiligen Eisenoxid-Pigmenten durch Behandlung mit einem Silan beschrieben wird.

Die Aufgabe der vorliegenden Erfindung ist es somit, biokompatible magnetische Nanopartikel mit einem hohen SAR-Wert in einem magnetischen Wechselfeld bereitzustellen, wobei die Beschichtung der Partikel aus einer stabilen siliziumhaltigen Hülle mit einer Dicke zwischen 0,5 und 10 nm, bevorzugt 1 nm bis 6 nm und weiter bevorzugt 3 nm, besteht. Die Stärke des für die Bestimmung der SAR eingesetzten magnetischen Wechselfeldes liegt bei einer Magnetfeldstärke von 4 kA/m und die Frequenz bei 100 kHz.

Die beschriebene Aufgabe wird durch das Herstellungsverfahren gemäß Anspruch 1, die Nanopartikel gemäß Anspruch 18 sowie die pharmazeutische Zusammensetzung gemäß Anspruch 25 gelöst.

Weitere vorteilhafte Ausgestaltungen ergeben sich aus den abhängigen Ansprüchen, den Beispielen, den Figuren und der Beschreibung.

Die vorliegende Erfindung betrifft biokompatible Nanopartikel mit einer stabilen siliziumhaltigen Hülle, die vorzugsweise eine Dicke zwischen 0,5 und 10 nm, weiter bevorzugt 1 nm bis 6 nm und weiter bevorzugt 2 nm bis 4 nm und insbesondere bevorzugt 3 nm hat und einem hohen SAR-Wert in einem magnetischen Wechselfeld, wobei die Stärke des magnetischen Wechselfeldes bei 4 kA/m und die Frequenz bei 100 kHz liegen.

Erfindungsgemäß lassen sich die Partikel mit hohem SAR-Wert durch ein Verfahren herstellen, welches die folgenden Schritte umfasst.
A1) Bereitstellung einer Zusammensetzung mindestens einer eisenhaltigen Verbindung A in mindestens einem organischen Lösungsmittel LM1,
B1) Erhitzen der Zusammensetzung für mindestens 10 Minuten auf eine Temperatur im Bereich von 50°C bis zu einer Temperatur von 50°C unterhalb der eigentlichen Reaktionstemperatur gemäß Schritt C1 der eisenhaltigen Verbindung A,
C1) Erhitzen der Zusammensetzung auf eine Temperatur zwischen 200°C und 400°C, wobei die Dauer der Heizphase mindestens 30 Minuten beträgt,
D1) Reinigung der erhaltenen Partikel mittels Soxhlet-Extraktion, wobei die Dauer der Extraktion zwischen 1 und 8 Stunden liegt,
E1) Suspendierung der gereinigten Nanopartikel in Wasser oder einer wässrigen Lösung einer Säure,
F1) Zugabe einer oberflächenaktiven Verbindung zur wässrigen Suspension erhalten gemäß Schritt E1),
H1) Reinigung der erhaltenen wässrigen Dispersion der Partikel,
I1) Herstellung einer Dispersion der Partikel gemäß Schritt H1) in einem Lösungsmittelgemisch aus Wasser und einem mit Wasser mischbaren Lösungsmittel
J1) Zugabe eines Alkoxysilans zur Dispersion der Partikel in dem Lösungsmittelgemisch gemäß Schritt I1),
K1) Reinigung der Partikel.

Die Schritte A1 bis K1 folgen in der Regel aufeinander, wobei nach dem Schritt A1 und vor Schritt B1 noch ein Schritt A2 und/oder nach Schritt B1 und vor Schritt C1 noch ein Schritt B2 folgen kann. Ebenso kann und nach Schritt C1, D1, E1, F1, G1, H1, I1, J1 oder K1 optional noch ein Oxidationsschritt C2, D2, E2, F2, G2, H2, 12, J2 oder K2 folgen. Der Schritt C2, D2, E2, F2, G2, H2, 12, J2 oder K2 wird hierin auch als Schritt X2 bezeichnet. Die zusätzlichen Schritte A2, B2 und/oder X2 sind optional und nicht essentiell für die Ausführung der Erfindung.

Ferner gehört es zum Durchschnittskönnen eines Fachmanns je nach gewählter Reaktionstemperatur und je nach gewählter eisenhaltiger Verbindung A oder je nach den gewählten anderen Komponenten die Reaktionsparameter anzupassen und zu optimieren. Die Dauer der Heizphase B1 der jeweiligen Reaktion kann ein Fachmann beispielsweise so optimieren, dass Partikel mit einer maximalen SAR entstehen. Die Dauer der Heizphase gemäß Schritt B1 beträgt mindestens 10 Minuten und es ist für einen Fachmann offensichtlich, dass sich die Heizphase mit steigender Temperatur verkürzt. Ebenso können bei dem erfindungsgemäßen Verfahren die Aufheizgeschwindigkeit, die Endtemperatur und die Haltedauer der Endtemperatur in Schritt C1 von einem Fachmann so angepasst werden, dass Partikel mit einer maximalen SAR entstehen.

Bei den Partikeln handelt es sich vorzugsweise um Nanopartikel, also Partikel mit einem Durchmesser im Nanometerbereich, wobei nach dem erfindungsgemäßen Verfahren auch Mikropartikel erhalten werden können.

Die eingesetzten eisenhaltigen Verbindungen A oder die eingesetzte eisenhaltige Verbindung A wird vorzugsweise aus der Gruppe ausgewählt, umfassend oder bestehend aus Eisen-Komplexverbindungen, Eisencarbonyl-Verbindungen, Eisen-Salze, insbesondere Eisensalze von gesättigten oder ungesättigten Fettsäuren, organische Eisenverbindungen und Eisen-Sandwich-Komplexe.

Als Eisencarbonylverbindung kann Eisendicarbonyl (Fe(CO)₂), Eisentetracarbonyl (Fe(CO)₄) oder Eisenpentacarbonyl (Fe(CO)₅) genannt werden und Beispiele für Eisen-Salze sind Eisendichlorid, Eisendibromid, Eisendifluorid, Eisendiiodid, Eisentrichlorid, Eisentribromid, Eisentrifluorid, Eisentriiodid, Eisen(II)sulfat, Eisen(III)sulfat, Eisenacetat, Eisenoxalat, Eisen(II)nitrat, Eisen(III)nitrat, Eisencarbonat, Eisen(II)hydroxid, Eisen(III)hydroxid, Eisenphosphat, Trieisendiphosphat. Ferrocen ist ein Beispiel für einen Eisen-Sandwich-Komplex und Eisenacetylacetonat für eine Eisen-Komplexverbindung. Als metallorganische Eisenverbindungen gelten z.B. Eisen(II)Acetat, Eisen(III)Acrylat, Eisen(III)Oleat, Eisenalkoxide wie Eisen(III)Ethoxid oder auch Eisencarbonyl-Verbindungen wie Acetylcyclobutadien-eisentricarbonyl, Butadien-eisentricarbonyl und Olefineisentetracarbonyl.

Als organisches Lösungsmittel LM1 können sämtliche hochsiedende Lösungsmittel verwendet werden. Bevorzugt sind Lösungsmittel aus der Gruppe umfassend oder bestehend aus: hochsiedende Amine, Alkane, Olefine, Alkohole oder Ether. Darüber hinaus können Monoether und Diether von Diolen (Alkandiolen) sowie Monoether, Diether und Triether von Triolen (Alkantriolen), Alkylenglykolmonoether, Alkylenglykoldiether, Ethylenglykolmonoether, Ethylenglykoldiether, Propylenglykolmonoether, Propylenglykoldiether, Glycerinmonoether, Glycerindiether, Glycerintriether und Glykol-Diether (Glymes) verwendet werden. Auch das Lösungsmittel L2 kann aus der vorgenannten Gruppe ausgewählt werden.

Besonders bevorzugte Lösungsmittel LM1 als auch LM2 sind Glykol-Diether (auch als "Glymes" bezeichnet) mit einem Siedepunkt von > 200°C. Zur Nanopartikel-Herstellung aus Eisen-Salzen (z.B. Chloriden) eignet sich auch Ethylenglykol. Grundsätzlich sollte der Siedepunkt des Lösungsmittels jedoch über 150°C, weiter bevorzugt über 175°C und insbesondere bevorzugt über 200°C liegen.

Die mindestens eine eisenhaltige Verbindung A wird in dem Lösungsmittel LM1 dispergiert, gelöst oder aufgeschlämmt und danach wird die erhaltene Zusammensetzung für mindestens 10 Minuten auf eine Temperatur im Bereich von 50°C bis zu einer Temperatur von 50°C unterhalb der eigentlichen Reaktionstemperatur gemäß Schritt C1 der eisenhaltigen Verbindung A erhitzt. Als eigentliche Reaktionstemperatur wird die Temperatur zur Partikelbildung verstanden, welche zwischen 200°C und 400°C liegt. Die Keimbildungstemperatur gemäß Schritt B1 liegt somit zwischen 50°C und maximal 350°C, jedoch stets mindestens 50°C unter der Temperatur gemäß Schritt C1. Vorzugsweise erfolgt somit das Erhitzen von einer oder mehrerer eisenhaltiger Verbindungen A in dem organischen Lösungsmittel LM1 oder dem Gemisch organischer Lösungsmittel LM1 auf eine Temperatur von ca. 50°C unterhalb der eigentlichen Temperatur zur Partikelbildung gemäß Schritt C1 der Verbindung A.

Diese der Partikelbildung vorgeschaltete Heizphase gemäß Schritt B1) dient der Bildung von so genannten Keimen, die danach eine definierte Partikelbildung ermöglichen. Die Dauer der Heizphase hat einen großen Einfluss auf die SAR der in Schritt C1) entstehenden Partikel, vorzugsweise Nanopartikel. Um Partikel bzw. Nanopartikel mit einem hohen SAR-Wert herzustellen, wird die erreichte Temperatur für mindestens 30 Minuten und insbesondere bevorzugt ca. 40 Minuten gehalten. Somit sollte die Zusammensetzung aus mindestens einer eisenhaltigen Verbindung A und mindestens einem Lösungsmittel LM1 für vorzugsweise 30 bis 50 Minuten auf die beschriebene Temperatur erwärmt werden.

Abhängig von der eingesetzten eisenhaltigen Verbindung A wird vorzugsweise eine Temperatur, die ca. 100°C bis 300°C, bevorzugt ca. 130°C bis 270°C, weiter bevorzugt ca. 150°C bis 250°C, noch weiter bevorzugt ca. 170°C bis 230°C, noch weiter bevorzugt 180°C bis 220°C, noch weiter bevorzugt ca. 190°C bis 210°C und insbesondere bevorzugt ca. 200°C unterhalb der eigentlichen Reaktionstemperatur zur Partikelbildung gemäß Schritt C1) liegt, angestrebt, wobei die angestrebte Temperatur nicht unter 70°C, bevorzugt nicht unter 90°C und insbesondere bevorzugt nicht unter 100°C liegen sollte. Bevorzugt wird die Temperatur bei der ersten Heizphase gemäß Schritt B1) auf 100°C bis 150° gehalten.

Um die Keimbildung zu beeinflussen oder zu begünstigen, können gemäß Schritt A2) Additive oder oberflächenaktive Verbindungen zugegeben werden. Die Begriffe "Additiv" oder "oberflächenaktive Verbindung" wie hierin verwendet stehen in dem Zusammenhang, dass die meisten Additive auch oberflächenaktive Verbindungen sind, dies jedoch nicht für alle Additive zwangsläufig gilt. Jede oberflächenaktive Verbindung kann daher als Additiv bezeichnet werden, wobei aber nicht jedes Additiv als oberflächenaktive Verbindung bezeichnet werden kann. Dabei kann es sich um Tenside, Silane, Si- oder Al-organische Verbindungen, Phosphine, gesättigte oder ungesättigte Fettsäuren, Amine, Diamine, Carbonsäuren und deren Salze, gesättigte und ungesättigte Fettsäuren oder auch um Polymere handeln. Beispiele für Polymere sind Polyvinylalkohol, Polyethylenglykol, Polyacrylsäure, Dextran, PLGA, Chitin, Fibrin, Heparin, Chitosan sowie Polyethylenimin.

Nach der Heizphase gemäß Schritt B1) erfolgt die eigentliche Partikelbildung in Schritt C1). Die in Schritt B1) entstandenen Partikel-Keime werden auf eine Temperatur zwischen 200°C und 400°C erhitzt, wobei die Dauer der Heizphase mindestens 30 Minuten beträgt.

Dabei bilden sich aus den entstandenen Partikel-Keimen und der überschüssigen eisenhaltigen Verbindung A eisenhaltige Partikel, vorzugsweise die eisenhaltigen Nanopartikel.

Es hat sich als vorteilhaft erwiesen, die Heizphase gemäß B1) nicht mit der gesamten Menge an eisenhaltiger Verbindung A zu beginnen und durchzuführen, sondern nach dem Keimbildungsschritt gemäß B1) mindestens eine weitere eisenhaltige Verbindung B in einem organischen Lösungsmittel L2 in einem Schritt B2) zuzusetzen.

Die mindestens eine eisenhaltige Verbindung B kann dabei aus der oben erwähnten Gruppe eisenhaltiger Verbindungen ausgewählt werden und kann identisch oder verschieden von der mindestens einen eisenhaltigen Verbindung A sein.

Gleiches gilt für das organische Lösungsmittel L2, welches aus der oben beschriebenen Gruppe der Lösungsmittel LM1 ausgewählt werden kann und identisch oder verschieden von dem Lösungsmittel LM1 ist, wobei bevorzugt ist, wenn Lösungsmittel LM1 und LM2 identisch sind.

Somit ist bevorzugt, wenn nach dem Keimbildungsschritt B1) neue eisenhaltige Verbindung B in vorzugsweise demselben Lösungsmittel (LM1 = LM2) zugesetzt wird und die so erhaltene Zusammensetzung gemäß C1) auf eine Temperatur bis 500°C vorzugsweise im Bereich von 200°C bis 400°C erhitzt wird. LM1 und LM2 haben vorzugsweise einen Siedepunkt von mindestens 200°C.

Hierbei werden nach Zugabe der mindestens einen eisenhaltigen Verbindung B in Lösungsmittel L2 die eigentlichen Partikel hergestellt. Zusammen mit der eisenhaltigen Verbindung B können auch weitere Additive der Zusammensetzung erhalten nach Schritt B1 zugesetzt werden. Auch bei den Additiven ist nicht zwingend, dass dieselben bereits in der Lösung vorhandenen Additive gewählt werden, wobei dies jedoch bevorzugt ist.

Auch hier kann wieder die Menge an zugegebener Eisenverbindung B, der Additive, sowie Art und Menge des Lösungsmittels L2 so von einem Fachmann angepasst werden, dass Partikel mit einer maximalen SAR entstehen.

Wie bereits ausgeführt, kann die Gesamtmenge an benötigter eisenhaltiger Verbindung jedoch auch bereits in Schritt A zugesetzt werden, so dass Schritt B2) bevorzugt jedoch nicht zwingend ist. Selbst dann, wenn nach der ersten Heizphase gemäß Schritt B1) keine weitere eisenhaltige Verbindung B mehr zugesetzt wird, kann dennoch als Schritt B2) weiteres Additiv zugesetzt werden, welches gleich dem bereits in der Zusammensetzung vorhandenen Additiv sein sollte. Als Schritt B2) kann somit nur Additiv oder nur eisenhaltige Verbindung B oder beides zusammen auf einmal oder nacheinander zugesetzt werden.

Die Dauer der 2. Heizphase gemäß Schritt C1) beträgt mindestens 30 Minuten und bevorzugt 1-30 Stunden, weiter bevorzugt 10-20 Stunden und besonders bevorzugt 15 Stunden.

Überraschend hat sich herausgestellt, dass die SAR durch Verlängerung der Heizphasen oder eben durch längere Heizphasen nochmals gesteigert werden kann, so dass lange Heizphasen und insbesondere die zusätzlichen Temperphasen bevorzugt sind. Insbesondere bei Schritt C1 ist eine Heizphase, die länger als 10 Stunden, bevorzugt länger als 14 Stunden ist bevorzugt.

Die nach dem Schritt D1 als D1* und/oder D2* optional folgende Temperphasen vermögen die SAR ebenfalls weiter zu steigern, und dauern daher vorzugsweise auch mehr als 10 Stunden, weiter bevorzugt mehr als 14 Stunden und insbesondere bevorzugt mehr als 18 Stunden. Eine Temperphase kann daher beispielsweise 1-30 Stunden, bevorzugt 10-25 Stunden, weiter bevorzugt 13-22 Stunden und insbesondere bevorzugt 15-20 Stunden dauern.

Die SAR der entstehenden Partikel kann durch Variation der Dauer der Heizphase B1), der Endtemperatur und der Haltedauer der Endtemperatur in Schritt C1), sowie der Menge an zugegebenen Eisenverbindungen bzw. Additiven in Schritt C1) angepasst werden, so dass Partikel mit einer maximalen SAR entstehen. Diese Parameter sind abhängig von der Art der eingesetzten Eisenverbindung und der Art des Lösungsmittels und der Additive. Die Heizphasen müssen demnach für jedes System angepasst werden, was von einem Fachmann jedoch aufgrund seiner Fachkenntnisse leicht durchgeführt werden kann.

Die SAR der erfindungsgemäß hergestellten Partikel liegt zwischen 10-40 W pro g Fe bei einer Magnetfeldstärke von 4 kA / m, bevorzugt zwischen 20-40 W pro g Fe bei einer Magnetfeldstärke von 4 kA / m, weiter bevorzugt zwischen 25-40 W pro g Fe bei einer Magnetfeldstärke von 4 kA / m und insbesondere bevorzugt zwischen 30-40 W pro g Fe bei einer Magnetfeldstärke von 4 kA / m und einer Frequenz des magnetischen Wechselfeldes von 100 kHz.

Im Folgenden werden einige Beispiele für Systeme zur Herstellung von erfindungsgemäßen Partikeln sowie die SAR-Werte der hergestellten Partikel angegeben. Die Beispiele (I) bis (VIII) ergeben erfindungsgemäße Partikel mit SAR-Werten von 20-40 W pro g Fe bei einer Magnetfeldstärke von 4 kA / m und einer Frequenz des magnetischen Wechselfeldes von 100 KHz. Die Angabe "Fe" betrifft den Gesamteisengehalt aus Fe°, Fe⁺² und Fe⁺³.

**Tabelle 1:**

| Beispiele für geeignete Komponenten zur Herstellung erfindungsgemäßer Partikel: | | | |
|---|---|---|---|
| Eisenhaltige Verbindung A | Lösungsmittel LM1 (Sdp.) | Beispiele für Additive | SAR [W/g Fe] |
| Eisenpentacarbonyl | Diethylenglykol-Dibutylether (256°C) | Fettsäuren, Tenside | 10-30 |
| Eisenpentacarbonyl | Dioctylether (287°C) | Fettsäuren, Tenside | 10-30 |
| Ferrocen | Diethylenglykoldibutytether (256°C) | Fettsäuren, Tenside, Amine | 10-30 |
| Eisenacetylacetonat | Trioctylamin (365°C) | Fettsäuren, Tenside, Amine | 15-30 |
| Eisenacetylacetonat | Ethylenglykol (197°C) | Diamine, Carbonsäuren, Polymere ohne Additiv | 15-30 |
| Eisenacetylacetonat | Triethylenglykol (291 °C | Diamine, Carbonsäuren, Polymere ohne Additiv | 15-30 |
| Eisen(III) Oleat | Polyglycol DME 500 (>250°C) | Fettsäuren, Tenside ohne Additiv | 15-35 |
| Eisen(III) Oleat | Trioctylamin (365°C) | Fettsäuren, Tenside | 15-35 |
| Eisen(II) Oleat | Polyglycol DME 500 (>250°C) | Fettsäuren, Tenside ohne Additiv | 15-30 |
| Eisen(II) Oleat | Trioctylamin (365°C) | Fettsäuren, Tenside | 15-35 |
| Eisen(III)Chlorid | Ethylenglykol (197°C) | Diamine, Carbonsäuren, Polymere ohne Additiv | 20-40 |
| Eisen(III)Chlorid | Triethylenglykol (291 °C) | Diamine, Carbonsäuren, Polymere | 20-35 |
| Eisen(III)Ethoxid | Polyglycol DME 500 (>250°C) | Diamine, Carbonsäuren, Polymere ohne Additiv | 10-25 |

In der vorgenannten Tabelle 1 bedeutet "ohne Additiv", dass die erfindungsgemäße Synthese mit den in der jeweiligen Spalte genannten Komponenten jedoch ohne Zugabe von Additiv durchgeführt worden ist. Die in der Tabelle 1 genannten Komponenten wurden gemäß Beispiel 1 und 3A oder 2 und 3A (Schritte A1 bis C2) umgesetzt und danach wurden alle Systeme entsprechend der Beispiele 4-6 als auch 4-7 weiter umgesetzt. Bei der Anwendung einer zusätzlichen Temperphase (Beispiel 7; Schritt D1* oder D2*) hat sich herausgestellt, dass die SAR um ca. 5 W/g Fe bei ca. 5 kA / m erhöht werden konnte. Die in der Tabelle 1 angegebenen SAR-Werte beziehen sich auf eine Magnetfeldstärke von 4 kA / m und eine Frequenz des Wechselfeldes von 100 KHz.

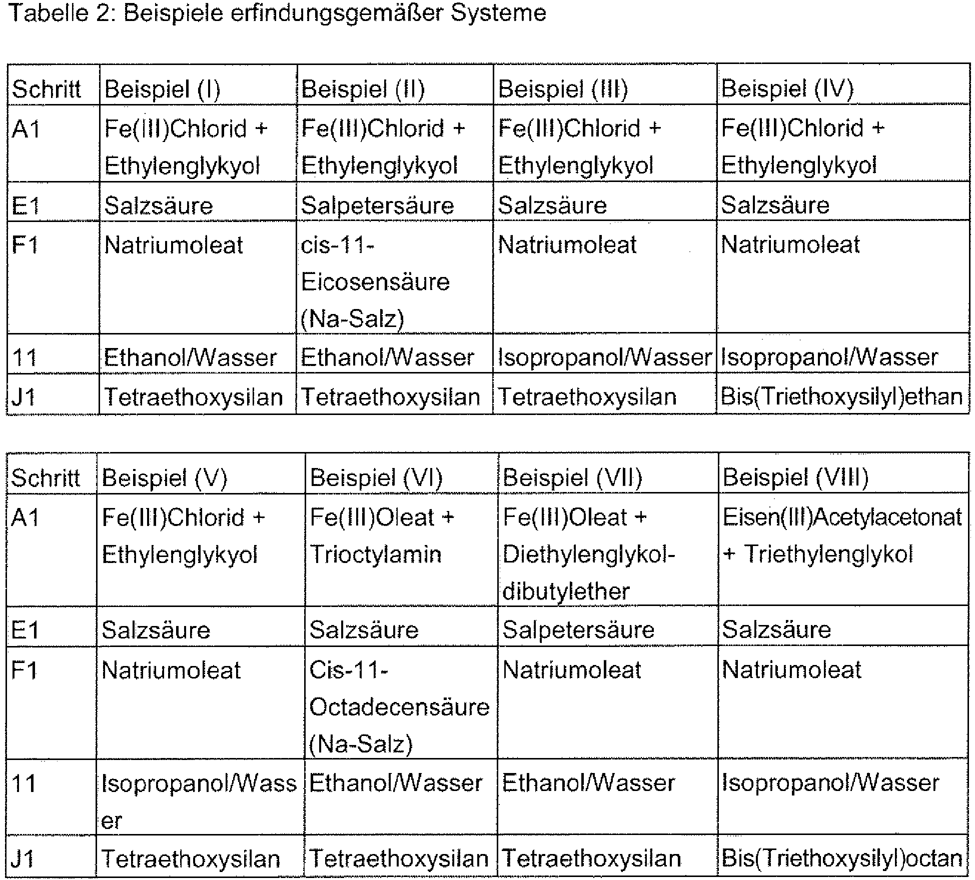

Die Phasen A) bis C) können wahlweise unter Normaldruck an Luft oder unter Schutzgasatmosphäre (Argon, Stickstoff) oder in einem Reaktionsautoklav unter Drucken bis zu 400 bar durchgeführt werden.

Nach dieser 2. Heizphase gemäß Schritt C1) kann sich eine Oxidationsphase X2) anschließen. Die Oxidationsphase X2) ist optional und muss nicht zwingend direkt nach Schritt C1 erfolgen, sondern kann auch nach einem der Schritte C1) bis K1) stattfinden. Die Partikel werden hierbei vorzugsweise durch Durchleitung von Luftsauerstoff oxidiert. Die Durchleitung von Luftsauerstoff erfolgt für 4 bis 24 Stunden, vorzugsweise 8 bis 16 Stunden und zudem bevorzugt bei 20 bis 50°C. Es können jedoch auch andere flüchtige oder destillativ entfernbare Oxidationsmittel wie beispielsweise Sauerstoff (rein), Wasserstoffperoxid oder auch organische Oxidationsmittel, wie z.B. Aminoxide eingesetzt werden. Bevorzugt ist somit, wenn sich nach einem der Schritte C1) bis K1) ein Oxidationsschritt X2) anschließt, wobei X eine variable für die Buchstaben C bis K ist, je nachdem, nach welchem Schritt die Oxidation erfolgt. Sollte die optionale Oxidation nach Schritt E1) erfolgen, so wird die Oxidation als Schritt E2) bezeichnet und sollte sie nach K1) erfolgen, dann wird der Oxidationsschritt eben mit K2) bezeichnet. Ferner kann der Oxidationsschritt auch mehrmals wiederholt werden oder ein weiterer Oxidationsschritt X2' kann nach einem weiteren Verfahrensschritt erfolgen, was möglich, jedoch nicht bevorzugt ist. Demnach konnte ein erfindungsgemäßes Verfahren einen ersten Oxidationsschritt X2 (z.B. F2) und einen zweiten Oxidationsschritt X2' (z.B. H2') umfassen. Bei Partikeln, welche sich bereits in einem teilweisen oder vollständigen oxidierten Zustand befinden, ist natürlich eine weitere Oxidation überflüssig. In der Regel verlaufen Oxidationen unter Luftatmosphäre von selbst, so dass ein zusätzlicher, d.h. zu der von alleine ablaufenden Oxidation zusätzlicher Oxidationsschritt X2 nicht erforderlich ist. Ein Oxidationsschritt X2 kann jedoch durchgeführt werden, auch wenn er nicht unbedingt nötig sein sollte, da sich herausgestellt hat, dass er auch nicht schadet.

Die gemäß Schritt C1 gebildeten Partikel, vorzugsweise Nanopartikel müssen gereinigt werden. Dieser Schritt ist essentiell und erfindungswesentlich. Die Umsetzung nicht gereinigter Partikel liefert keine erfindungsgemäßen Partikel mit guter Dispergierbarkeit in Wasser und letztendlich hoher SAR. Diese Reinigung erfolgt gemäß Schritt D1) durch eine Soxhlet-Extraktion, wobei die Dauer der Extraktion zwischen 1 und 8 Stunden liegt, bis eine Dispergierbarkeit der Partikel in unpolaren Lösungsmitteln nicht mehr gewährleistet ist. Wie überraschend gefunden wurde, ist es für die spätere Dispergierung in Wasser [Schritt F1)] essentiell, dass das Additiv und insbesondere die oberflächenaktive Substanz aus Schritt A2) und/oder B2) möglichst vollständig von den Partikeln abgewaschen wird, d.h. weitgehend wieder entfernt werden. Unter dem Begriff "möglichst vollständig" bzw. "weitgehend" wird eine Entfernung der Additive im Bereich von 70-100% vorzugsweise bis zu 90% verstanden. Die Additive werden also zu mehr als 70%, bevorzugt mehr als 80%, weiter bevorzugt mehr als 90% und insbesondere bevorzugt mehr als 95% wieder entfernt, insbesondere von den Partikeln entfernt. Die vorgenannten Prozentangaben beziehen sich auf das an den Partikeln haftende Additiv. Freies Additiv, d.h. in der Lösung frei schwimmendes und nicht an Partikel adsorbiertes Additiv kann durch Zentrifugation weitgehend vollständig, d.h. zu >95%, bevorzugt zu >98% entfernt werden. Die Menge an verbleibendem an den Partikeln haftendem Additiv kann beispielsweise durch Elementaranalyse oder IR-Spektroskopie bestimmt werden. Die Prozentangabe bezieht sich dabei auf das Gewicht (Gew.-%). Das nicht an die Partikel haftende Additiv wird mittels Zentrifugation entfernt und das an den Partikeln haftende Additiv kann vorzugsweise durch eine Soxhlet-Extraktion der entstandenen Partikel erreicht werden, wobei aber auch eine durch Ultraschall-unterstutzte Extraktion zum Einsatz kommen kann. Zu diesem Zweck werden die Nanopartikel zunächst durch eine Zentrifugation absepariert, bevor der weitere Reinigungsschritt folgt.

Bei dem für die Soxhlet-Extraktion verwendeten Lösungsmittel kann es sich um gängige polare organische Lösungsmittel, wie Alkohole, Ketone, Ether oder Ester handeln. Bevorzugt verwendet werden Aceton, Ethylacetat oder Ethanol.

Die Dauer der Extraktion liegt zwischen 1 und 8 Stunden, bevorzugt zwischen 2 und 6 Stunden und insbesondere bevorzugt bei ca. 4 Stunden. Entscheidend ist, dass die eisenhaltigen Partikel vorzugsweise Nanopartikel nach der Extraktion nicht mehr in unpolaren Lösungsmitteln, wie z.B. Toluol, Xylol oder Hexan dispergierbar sind. Sollte dies noch der Fall sein, muss die Extraktionsdauer angepasst werden. Das so gereinigte Nanopartikel-Pulver wird im Vakuum getrocknet.

Nach dem Schritt D1 können sich mehrere "Temperphasen" anschließen, um die Kristallinität der Partikel zu erhöhen. Diese Temperphasen können in hochsiedenden Lösungsmitteln bei bis zu 400°C für mehrere Stunden durchgeführt werden. Als hochsiedende Lösungsmittel werden solche bezeichnet, die einen Siedepunkt von mindestens 200°C bevorzugt mindestens 300°C aufweisen. Hierbei kann der Temperprozess, sowohl an Luft als auch unter Schutzgas (z.B. Argon) stattfinden. Bei Temperaturen bis ca. 200 bis 250°C wird vorzugsweise ohne Schutzgas gearbeitet und bei Temperaturen ab ca. 200 bis 250°C wird vorzugsweise unter Schutzgas gearbeitet. Alternativ können die Nanopartikel auch als Pulver (ohne Lösungsmittel) bei Temperaturen bis zu 1000°C unter Schutzgasatmosphäre getempert werden. Bevorzugte Schutzgase sind Argon oder CO₂/H₂-Gemische. Dieser mindestens eine Temperschritt schließt sich als Schritt D1* an den Schritt D1 oder als D2* an den Oxidationsschritt D2* an.

Die Oxidation gemäß Schritt X2 mit X = C oder D oder E oder F oder G oder H oder I oder J oder K kann vorzugsweise durch Suspendieren der Partikel in 0,5 bis 2M HNO₃, vorzugsweise 1 M HNO₃, Zugabe von Fe(NO₃)₃ und anschließendem Kochen unter Rückfluss durchgeführt werden. Das Verhältnis von Fe(NO₃)₃ zu FeOₓ oder allgemein das Verhältnis von Fe(III) zu FeOₓ beträgt dabei vorzugsweise 1:2. Dieser Oxidationsprozess wirkt sich vorteilhaft auf die SAR der Partikel aus und ist daher bevorzugt. Es sei angemerkt, dass dieser Schritt nicht auf Fe(NO₃)₃ beschränkt ist und auch andere Fe(III)-Salze eingesetzt werden können wie z.B. FeCb, FePO₄ usw.

Nun erfolgt gemäß, den Schritten E1) und F1) die Dispergierung der gereinigten Partikel bzw. Nanopartikel in Wasser durch reversible Beschichtung mit einer oberflächenaktiven Verbindung.

Bei diesem Schritt wird das gereinigte Partikel-Pulver bzw. Nanopartikel-Pulver in Wasser suspendiert, wobei eine hydrophile Schicht so angekoppelt wird, dass eine spätere Entfernung dieser Schicht leicht möglich ist. Bei der Beschichtung wird anfänglich ein Feststoffgehalt (Eisenoxid) von vorzugsweise 2-20%, weiter bevorzugt 3-12%, noch weiter bevorzugt 5%-8%, noch weiter bevorzugt 6%-7% und insbesondere bevorzugt ca. 6,5% eingestellt. Um eine feine Dispergierung der Partikel zu erreichen, kann vor der Zugabe der oberflächenaktiven Verbindung eine Säure vorzugsweise eine Mineralsäure gemäß Schritt F1) wie z.B. Salzsäure oder Salpetersäure zugegeben werden, so dass sich ein pH-Wert von 2-6, vorzugsweise 3-5 und insbesondere bevorzugt von ca. 4 ergibt.

Als Säure können vorzugsweise Mineralsäuren wie beispielsweise Salzsäure, Phosphorsäure, Schwefelsäure, Bromwasserstoffsäure, Borsäure oder Salpetersäure gewählt werden. Wichtig ist nur, dass eine Säure, vorzugsweise eine anorganische Säure eingesetzt wird, welche nicht irreversibel an die Partikeloberfläche bindet. Es hat sich bei den Versuchen gezeigt, dass Mineralsäuren bevorzugt sind und Aminosäuren als auch Carbonsäuren vermieden werden sollten. Folgende Säuren sind aber grundsätzlich bei dem erfindungsgemäßen Verfahren einsetzbar: Sulfonsäure, salpetrige Säure, Perchlorsäure, Methansulfonsäure, Ethansulfonsäure, Hydroxyethansulfonsäure, Ethylensulfonsäure, p-Toluolsulfonsäure, Naphthylsulfonsäure, Naphthylaminsulfonsäure, Sulfanilsäure und Camphersulfonsäure.

Ist der pH-Wert der wässrigen Lösung mittels Säure bzw. Mineralsäure eingestellt, so erfolgt die Zugabe der mindestens einen oberflächenaktiven Verbindung gemäß Schritt F1). Die mindestens eine oberflächenaktive Verbindung wird bevorzugt aus der Gruppe ausgewählt umfassend oder bestehend aus Salzen von gesättigten und besonders bevorzugt von ungesättigten Fettsäuren. Darüber hinaus konnten Tenside oder Polymere, wie z.B. Polyvinylalkohol, Polyethylenglykol, Polyacrylsäure, Dextran, PLGA, Chitosan, Polyethylenimin eingesetzt werden.

Beispiele für gesättigte Fettsäuren sind: Essigsäure, Propionsäure, Buttersäure, Capronsäure, Caprylsäure, Caprinsäure, Laurinsäure, Myristinsäure, Palmitinsäure, Margarinsäure, Stearinsäure, Arachinsäure, Behensäure und Lignocerinsäure.

Als Beispiele für die bevorzugten ungesättigten Fettsäuren oder deren Salze können beliebige Fettsäuren genannt werden wie beispielsweise cis-9-Tetradecensäure (Myristoleinsäure), cis-9-Hexadecensäure (Palmitoleinsäure), cis-6-Octadecensäure (Petroselinsäure), cis-9-Octadecensäure (Ölsäure), cis-11-Octadecensäure (Vaccensäure), cis-9-Eicosensäure (Gadoleinsäure), cis-11-Eicosensäure (Gondoinsäure), cis-13-Docosensäure (Erucinsäure), cis-15-Tetracosensäure (Nervonsäure), t9-Octadecensäure (Elaidinsäure), t11-Octadecensäure (t-Vaccensäure), t3-Hexadecensäure, 9,12-Octadecadiensäure (Linolsäure), 6,9,12-Octadecatriensäure (γ-Linolsäure), 8,11,14-Eicosatriensäure (Dihomo-γ-linolensäure), 5,8,11,14-Eicosatetraensäure (Arachidonsäure), 7,10,13,16-Docosatetraensäure, 4,7,10,13,16-Docosapentaensäure, 9,12,15-Octadecatriensäure (α-Linolensäure), 6,9,12,15-Octadecatetraensäure (Stearidonsäure), 8,11,14,17-Eicosatetraensäure, 5,8,11,14,17-Eicosapentaensäure (EPA), 7,10,13,16,19-Docosapentaensäure (DPA), 4,7,10,13,16,19-Docosahexaensäure (DHA), 5,8,11-Eicosatriensäure (Meadsäure), 9c 11t 13t Eleostearinsäure, 8t 10t 12c Calendinsäure, 9c 11t 13cCatalpinsäure, 4, 7, 9,11, 13, 16, 19 Docosaheptadecansäure (Stellaheptaensäure), Taxolsäure, Pinolensäure, Sciadonsäure, 6-Octadecinsäure (Taririnsäure), t11-Octadecen-9-insäure (Santalbin-oderXimeninsäure), 9-Octadecinsäure (Stearolinsäure), 6-Ociadecen-9-insäure (6,9-Octadeceninsäure), t10-Heptadecen-8-insäure (Pyrulinsäure), 9-Octadecen-12-insäure (Crepeninsäure), t7,t11-Octadecadiene-9-insäure (Heisterinsäure), t8,t10-Octadecadiene-12-insäure, 5,8,11,14-Eicosatetrainsäure (ETYA) und t8,t10-Octadecadiene-12-insäure. Die Salze der Fettsäuren werden bevorzugt mit Alkali- und Erdalkaliionen gebildet.

Das Massenverhältnis von Nanopartikeln zu oberflächenaktiver Verbindung betragt vorzugsweise 1 : 0,02 bis 1:10, weiter bevorzugt 1 : 0,1 bis 1 : 2 und insbesondere bevorzugt 1 : 0,5.

Nach der Zugabe der oberflächenaktiven Substanz wird die Suspension gemäß Schritt G1) vorzugsweise für mindestens 30 Minuten mit Ultraschall behandelt.

Die Suspension wird dann vorzugsweise bei einer Temperatur von 30°C bis 70°C, weiter bevorzugt 50°C bis 60°C, insbesondere bevorzugt bei 40°C für ca. 2 Stunden gerührt. Danach erfolgt eine Reinigung gemäß Schritt I1). Nicht-dispergierte Partikel werden vorzugsweise durch eine Zentrifugation (1000 U/min) abgetrennt.

Die Partikel-Dispersion muss unmittelbar nach der Beschichtung von überschüssiger oberflächenaktiver Substanz befreit werden. Diese Reinigung kann durch eine Dialyse oder eine Extraktion mit Diethylether erfolgen. Alternativ können die Partikel durch eine Ultrazentrifuge abzentrifugiert und mit Wasser und einer Mischung aus Wasser und Diethylether gewaschen werden.

Danach erfolgt der Austausch der Fettsäure basierten Partikel-Beschichtung gegen eine siliziumhaltige biokompatible Hülle gemäß Schritt I1) und J1).

Zum Austausch der Hülle werden die Partikel gemäß Schritt I1) in einem Gemisch aus Wasser und mindestens einem mit Wasser mischbaren Lösungsmittel dispergiert. Als mit Wasser mischbare Lösungsmittel werden Alkohole, Polyole, Tetrahydrofuran (THF), Dimethylformamid (DMF), Dimethylacetamid, Dimethylsulfoxid (DMSO), Aceton, Essigsäure, Ameisensäure, Ameisensäuremethylester, Ameisensäureethylester, Essigsäuremethylester, Essigsäureethylester und andere bezeichnet.

Insbesondere bevorzugt sind jedoch Alkohole. Der Alkohol kann bevorzugt aus der Gruppe ausgewählt werden, umfassend oder bestehend aus: Methanol, Ethanol, Propanol, Isopropanol, Ethylenglykol, wobei Ethanol bevorzugt ist.

Das Mischungsverhältnis von Wasser zu Alkohol bzw. Wasser zu Ethanol betragt vorzugsweise 1:1 bis 1:5 und insbesondere bevorzugt 1:3, damit die Entfernung der Fettsäure-Hülle und der Austausch gegen die siliziumhaltige Hülle parallel stattfinden kann.

Zudem ist bevorzugt, wenn das Alkohol-Wasser-Gemisch 1-5 Gew.-%, weiter bevorzugt 1-3 Gew.-% und insbesondere bevorzugt 1,5 Gew.-% eines Amins, vorzugsweise eines primären Amins und insbesondere bevorzugt Ammoniak enthält.

Zeitnah zur Zugabe der Nanopartikel-Dispersion zum Lösungsmittelgemisch und insbesondere zum Wasser-Alkohol-Gemisch und vorzugsweise zum Wasser-Ethanol-Gemisch gemäß Schritt I1) muss dann ein geeignetes Alkoxysilan zugeben werden. Die Zugabe des Alkoxysilans sollte unter Ultraschall-Behandlung stattfinden. Geeignete Alkoxysilane sind sämtliche Tetraalkoxysilane, wie z.B. Tetramethoxysilan und Tetraethoxysilan, sowie Trialkoxysilane, Dialkoxysilane und Monoalkoxysilane, die vorzugsweise zusätzlich eine über eine Si-C Bindung gekoppelte funktionelle Gruppe, wie z.B. Aminogruppe, Thiolgruppe und/oder Epoxygruppe tragen.

Damit der Austausch der Hüllbeschichtungen reibungslos funktioniert, sollte das molare Verhältnis von Eisen zum Alkoxysilan 1:1 bis 1:5 und bevorzugt 1:3 betragen.

Nach Zugabe der Reaktanden wird die Dispersion gemäß Schritt J1) für 1-8 Stunden, bevorzugt 3-5 Stunden und insbesondere bevorzugt 4 Stunden mit Ultraschall behandelt. Die Aufreinigung der Partikel erfolgt danach vorzugsweise durch Dialyse gegen Wasser. Alternativ kann die Aufreinigung durch Abzentrifugieren der Partikel bei einer hohen g-Zahl und Waschen des Niederschlags mit Reinstwasser erfolgen.

Ferner betrifft die vorliegende Erfindung Partikel und vorzugsweise Nanopartikel, welche nach dem hierin offenbarten Verfahren erhalten werden können.

Die erfindungsgemäßen eisenhaltigen Nanopartikel selbst sind vorzugsweise ferromagnetisch, ferrimagnetisch oder superparamagnetisch. Derartige Partikel bzw. Nanopartikel können durch ein magnetisches Wechselfeld erwärmt werden. Eine Erwärmung des die Partikel bzw. Nanopartikel enthaltenden Gewebes auf über 50°C ist möglich, da die Partikel bzw. Nanopartikel erfindungsgemäß hohe SAR-Werte besitzen.

Die erfindungsgemäß hergestellten eisenhaltigen Partikel besitzen einen SAR-Wert von10-40 W pro g Fe bei einer Magnetfeldstärke von 4 kA/m und einer Frequenz eines magnetischen Wechselfeldes von 100 kHz.

Die Partikel weisen vorzugsweise einen Durchmesser von weniger als 500 nm auf. Vorzugsweise besitzen die Nanopartikel einen durchschnittlichen Durchmesser in dem Größenbereich von 1-100 nm und insbesondere bevorzugt im Bereich von 15-30 nm.

Die stabile siliziumhaltige Hülle der Nanopartikel hat eine Dicke zwischen 0,5 und 10 nm, bevorzugt 3 nm.

Die siliziumhaltige Hülle kann durch weitere Alkoxysilane funktionalisiert werden, um die Eigenschaften der Partikel zu modifizieren. Dabei handelt es sich vorzugsweise um Trialkoxysilane, die eine über eine Si-C-Bindung gebundene funktionelle Gruppe tragen. Beispiele hierfür sind (3-acryloxypropyl)Trimethoxysilan, Triethoxysilylbutyraidehyd, 3-Aminopropyltriethoxysilan, 3-Isocyanatopropyltriethoxy-silan. Die Trialkoxysilane können aber auch über Si-C gebundene Polyethylenglykol-Seitenketten unterschiedlicher Länge tragen. Ein Beispiel hierfür ist 2-[methoxy(Polyethylenoxy)Propyl]Trimethoxysilan.

Die erfindungsgemäßen eisenhaltigen Partikel können für den Bereich der Medizin Verwendung finden und beispielsweise in wässriger Lösung injiziert werden. Die erfindungsgemäßen eisenhaltigen Partikel können zur Behandlung und Prophylaxe von proliferativen Erkrankungen, Krebs, Tumoren, Rheuma, Arthritis, Arthrose und bakteriellen Infektionen eingesetzt werden.

Des Weiteren betrifft die vorliegende Erfindung pharmazeutische Zusammensetzungen, welche die erfindungsgemäßen Nanopartikel enthalten.

Bei diesen pharmazeutischen Zusammensetzungen handelt es sich insbesondere um Infusion- oder Injektionslösungen. Derartige Lösungen der Nanopartikel in beispielsweise physiologischer Kochsalzlösung sind für die interstitielle bzw. intratumorale Applikation geeignet. Eine intraarterielle oder intravenöse Applikation ermöglicht ferner eine systemische, den ganzen Körper betreffende Therapiemöglichkeit für nicht solide und/oder metastasenbildende Tumorarten.

Weitere bevorzugte pharmazeutische Zusammensetzungen sind Pulver, Inhalationspulver sowie Lyophilisate enthaltend die erfindungsgemäßen eisenhaltigen Partikel.

Die erfindungsgemäßen Nanopartikel und pharmazeutischen Zusammensetzungen können bevorzugt für die Behandlung als auch zur Prophylaxe von Krankheiten eingesetzt werden, welche sich durch entartete Zellspezies oder körperfremde Zellen auszeichnen und bei denen die Eigenschaften der erfindungsgemäßen Nanopartikel- ausgenutzt werden können, zwischen fremden Zellen bzw. entarteten Zellen und gesunden körpereigenen Zellen zu diskriminieren. Als entartete Zellen gelten insbesondere Krebszellen bzw. in ihrer Proliferation gestörte Zellen als auch stenotisches oder restenotisches Gewebe. Als körperfremde Zellen können insbesondere Bakterien genannt werden.

Demzufolge können die erfindungsgemäßen Nanopartikel und die Nanopartikel enthaltenden pharmazeutischen Zusammensetzungen zur Prophylaxe und Behandlung von Tumoren, Karzinomen und Krebs eingesetzt werden.

Als Beispiele für Krebs- und Tumorarten, wo die erfindungsgemäßen Nanopartikel eingesetzt werden können, sind zu nennen: Adenokarzinome, Aderhautmelanom, Akute Leukämie, Akustikusneurinom, Ampullenkarzinom, Analkarzinom, Astrozytome, Basaliom, Bauchspeicheldrüsenkrebs, Bindegewebstumor, Blasenkrebs, Bronchialkarzinom, Nicht-kleinzelliges Bronchialkarzinom, Brustkrebs, Burkitt-Lymphom, Corpuskarzinom, CUP-Syndrom, Dickdarmkrebs, Dünndarmkrebs, Dünndarmtumore, Eierstockkrebs, Endometriumkarzinom, Ependymom, Epithel-Krebsarten, Ewing-Tumoren, Gastrointestinale Tumoren, Gallenblasenkrebs, Gallenkarzinome, Gebärmutterkrebs, Gebärmutterhalskrebs, Glioblastome, Gynäkologische Tumoren, Hals-, Nasen- und Ohrentumoren, Hämatologische Neoplasien, Haarzell-Leukämie, Harnröhrenkrebs, Hautkrebs, Hirntumoren (Gliome), Hirnmetastasen, Hodenkrebs, Hypophysentumor, Karzinoide, Kaposi-Sarkom, Kehlkopfkrebs, Keimzellentumor, Knochenkrebs, kolorektales Karzinom, Kopf-Hals-Tumore (Tumore des Hals-, Nasen- und Ohrenbereichs), Kolonkarzinom, Kraniopharyngeome, Krebs im Mundbereich und auf der Lippe, Leberkrebs, Lebermetastasen, Leukämie, Lidtumor, Lungenkrebs, Lymphdrüsenkrebs (Hodgkin/Non-Hodgkin), Lymphome, Magenkrebs, Malignes Melanom, malignes Neoplasma, Malignome des Magen-Darm-Traktes, Mammakarzinom, Mastdarmkrebs, Medulloblastome, Melanom, Meningeome, Morbus Hodgkin, Mycosis fungoides, Nasenkrebs, Neurinom, Neuroblastom, Nierenkrebs, Nierenzellkarzinome, Non-Hodgkin-Lymphome, Oligodendrogliom, Ösophaguskarzinom, osteolytische Karzinome. u. osteoplastische Karzinome, Osteosarkom, Ovarial-Karzinom, Pankreaskarzinom, Peniskrebs, Plasmozytom, Plattenepithelkarzinome des Kopfes und Halses, Prostatakrebs, Rachenkrebs, Rektumkarzinom, Retinoblastom, Scheidenkrebs, Schilddrüsenkarzinom, Schneeberger Krankheit, Speiseröhrenkrebs, Spinaliom, T-Zell-Lymphom (Mycosis fungoides), Thymom, Tubenkarzinom, Tumoren des Auges, Urethrakrebs, Urologische Tumoren, Urothelkarzinom, Vulvakrebs, Warzenbeteiligung, Weichteiltumoren, Weichteilsarkom, Wilms Tumor, Zervixkarzinom und Zungenkrebs.

Bevorzugt sind insbesondere solide Tumoren. Ferner sind bevorzugt Prostatakarzinome, Gehirntumore, Sarkome, Zervix-Karzinome, Ovarialkarzinome, Mammakarzinome, Bronchialkarzinome, Melanome, Kopf-Hals-Tumore, Ösophaguskarzinome, Rektumkarzinome, Pankreas-, Blasen- und Nierenkarzinome, Metastasen der Leber, des Gehirns und in Lymphknoten.

Insbesondere bevorzugt ist ferner die Anwendung und der Einsatz der erfindungsgemäßen Nanopartikel zusammen mit der konventionellen Hyperthermie, Strahlentherapie und/oder zusammen mit der herkömmlichen Chemotherapie.

Ferner konnte gefunden werden, dass die erfindungsgemäßen magnetischen und vorzugsweise superparamagnetischen Partikel die Aktivität von Antikrebsmitteln steigern und zudem deren Nebenwirkungen verringern.

Somit können die erfindungsgemäß hergestellten Partikel bevorzugt zusammen mit Antikrebsmitteln, also mit zytotoxischen und/oder zytostatischen Verbindungen, d.h. chemische Verbindungen mit zytotoxischen und/oder zytostatischen Eigenschaften verwendet werden. Beispiele für Antikrebsmittel sind unter anderem Alkylierungsmittel, Antibiotika mit zytostatischen Eigenschaften, Antimetabolite, Mikrotubuli-lnhibitoren und Topoisomerase-Inhibitoren, Platin-enthaltende Verbindungen und andere Zytostatika wie beispieisweise Asparaginase, Tretinoin, Alkaloide, Podophyllotoxine, Taxane und Miltefosin®, Hormone, Immunmodulatoren, monoklonale Antikörper, Signaltransduktoren (Signaltransduktionsmolekule), Kinaseinhibitoren und Zytokine.

Als Beispiele für Alkylierungsmittel können unter anderem Chlorethamin, Cyclophosphamid, Trofosfamide, Ifosfamid, Melphalan, Chlorambucil, Busulfan, Thiotepa, Carmustin, Lomustin, Dacarbazin, Procarbazin, Temozolomid, Treosulfan, Estramustin und Nimustin genannt werden.

Beispiele für Antibiotika mit zytostatischen Eigenschaften sind Daunorubicin als auch liposomales Daunorubicin, Doxorubicin (Adriamycin), Dactinomycin, Mitomycin C, Bleomycin, Epirubicin (4-Epi-Adriamycin), Idarubicin, Dactinomycin, Mitoxantron, Amsacrin und Actinomycin D.

Methotrexat, 5-Fluorouracil, 6-Thioguanin, 6-Mercaptopurin, Fludarabin, Cladribin, Pentostatin, Gemcitabin, Cytarabin, Azathioprin, Raltitrexed, Capecitabin, Cytosinarabinosid, Tioguanin und Mercaptopurin können als Beispiele für Antimetabolite (antimetabolische Wirkstoffe) angeführt werden.

Zu der Klasse der Alkaloide und Podophyllotoxine gehören unter anderem Vincristin, Vinblastin, Vindesin, Etoposid als auch Teniposid. Des Weiteren können Platin-enthaltende Verbindungen erfindungsgemäß eingesetzt werden. Als Platin-enthaltende Verbindungen seien beispielsweise Cisplatin, Carboplatin und Oxaliplatin genannt. Zu den Mikrotubuli-Inhibitoren zählen beispielsweise Alkaloide wie beispielsweise Vinca-Alkaloide (Vincristin, Vinblastin, Vindesin, Venorelbin) und Paclitaxel (Taxol®) sowie Derivate des Paclitaxel. Als Topoisomerase-Inhibitoren können beispielsweise Etoposid, Teniposid, Camptothecin, Topotecan und Irinotecan genannt werden.

Paclitaxel und Docetaxel sind Beispiele für die Verbindungsklasse der Taxane und zu den anderen zytostatischen Wirkstoffen (anderen Zytostatika) zählen beispielsweise Hydroxycarbamide (Hydroxyurea), Imatinib, Miltefosin®, Amsacrin, Topotecan (Topoisomerase-I-Inhibitor), Pentostatin, Bexaroten, Tretinoin und Asparaginase. Vertreter der Verbindungsklasse der monoklonalen Antikbrper sind unter anderem Trastuzumab (auch bekannt als Herceptin®), Alemtuzumab (auch bekannt als MabCampath®) und Rituximab (auch bekannt als MabThera®). Vertreter der Kinaseinhibitoren sind Sorafenib (Nexavar)® und Sunitinib (Sutent®).

Beispiele für Hormone sind Glucocorticoide (Prednison), Oestrogene (Fosfestrol, Estramustin), LHRH (Buserelin, Goserelin, Leuprorelin, Triptorelin), Flutamid, Cyproteronacetat, Tamoxifen, Toremifen, Aminoglutethimid, Formestan, Exemestan, Letrozol und Anastrozol. Zu den Klassen der Immunmodulatoren, Zytokine, Antikbrper und Signaltransduktoren zählen lnterleukin-2, Interferon-cc, Erythropoietin, G-CSF, Trastuzumab (Herceptin®), Rituximab (MabThera®), Efitinib (fressa®), Ibritumomab (Zevalin®), Levamisol sowie Retinoide.

Somit betrifft die vorliegende Erfindung auch Kombinationen aus den erfindungsgemäß hergestellten Partikeln mit mindestens einem Antikrebsmittel wie beispielsweise Actinomycin D, Aminoglutethimid, Amsacrin, Anastrozol, Antagonisten von Purin- und Pyrimidin-Basen, Anthracycline, Aromatasehemmer, Asparaginase, Antiöstrogene, Bexaroten, Bleomycin, Buselerin, Busulfan, Camptothecin-Derivate, Capecitabin, Carboplatin, Carmustin, Chlorambucil, Cisplatin, Cladribin, Cyclophosphamid, Cytarabin, Cytosinarabinosid, alkylierende Cytostatika, Dacarbazin, Dactinomycin, Daunorubicin, Docetaxel, Doxorubicin (Adriamycin), Doxorubicin lipo, Epirubicin, Estramustin, Etoposid, Exemestan, Fludarabin, Fluorouracil, Folsäureantagonisten, Formestan, Gemcitabin, Glucocorticoide, Goselerin, Hormone und Hormonantagonisten, Hycamtin, Hydroxyharnstoff, Idarubicin, Ifosfamid, Imatinib, Irinotecan, Letrozol, Leuprorelin, Lomustin, Melphalan, Mercaptopurin, Methotrexat, Miltefosin, Mitomycine, Mitosehemmstoffe, Mitoxantron, Nimustine, Oxaliplatin, Paclitaxel, Pentostatin, Procarbazin, Tamoxifen, Temozolomid, Teniposid, Testolacton, Thiotepa, Tioguanin, Topoisomerase-Inhibitoren, Topotecan, Treosulfan, Tretinoin, Triptorelin, Trofosfamide, Vinblastin, Vincristin, Vindesin, Vinorelbin, zytostatisch wirksame Antibiotika sowie pharmazeutische Zusammensetzungen enthaltend die vorgenannten Kombinationen.

Die vorgenannten Wirkstoffe können nicht nur in Kombination mit den erfindungsgemäßen Partikeln eingesetzt sondern auch kovalent an die Partikel vorzugsweise Nanopartikel gebunden werden, um so noch leichter in Krebszellen eingeschleust zu werden.

Ein weiterer Aspekt ist somit auf Partikel erhältlich nach dem erfindungsgemäßen Verfahren gerichtet, wobei eine therapeutisch wirksame Substanz kovalent an das Partikel bzw. Nanopartikel gebunden ist. Als therapeutisch wirksame Substanzen können anti-proliferative, anti-migrative, anti-angiogene, anti-thrombotische, antiinflammatorische, anti-phlogistische, zytostatische, zytotoxische, anti-koagulative, anti-bakterielle, anti-virale und/oder anti-mykotische Wirkstoffe gewählt werden, wobei anti-proliferative, anti-migrative, anti-angiogene, zytostatische und/oder zytotoxische Wirkstoffe sowie Nukleinsäuren, Aminosäuren, Peptide, Proteine, Kohlenhydrate, Lipide, Glycoproteine, Glycane oder Lipoproteine mit anti-proliferativen, anti-migrativen, anti-angiogenen, anti-thrombotischen, anti-inflammatorischen, anti-phlogistischen, zytostatischen, zytotoxischen, anti-koagulativen, anti-bakteriellen, anti-viralen und/oder anti-mykotischen Eigenschaften bevorzugt sind. Darüber hinaus können diese Substanzen auch Radiosensitizer oder Sensitizer oder Verstärker anderer auch kombinierter konventioneller Krebsbehandlungsmethoden sein oder solche Sentitizer enthalten.

Die Anbindung der therapeutisch wirksamen Substanzen kann beispielsweise über Hydroxygruppen, Aminogruppen, Carbonylgruppen, Thiolgruppen oder Carboxylgruppen erfolgen, je nachdem, welche funktionelle Gruppen der jeweilige Wirkstoff trägt.

Hydroxygruppen werden vorzugsweise als Ester, Acetal oder Ketal gebunden, Thiogruppen vorzugsweise als Thioester, Thioacetal oder Thioketal, Aminogruppen vorzugsweise als Amide und teilweise auch als Imine (Schiffsche Basen), Carboxylgruppen vorzugsweise als Ester oder Amide und Carbonylgruppen vorzugsweise als Ketale. Ferner ist die Funktionalisierung der Oberfläche der Nanopartikel bekannt, so dass nach bekannten Verfahren Aminogruppen, Hydroxygruppen, Carboxylgruppen oder Carbonylgruppen auf der Oberfläche der Nanopartikel erzeugt werden können.

Eine weitere Beschichtung der aktivierbaren Nanopartikel-Wirkstoff-Konjugate (z.B. durch Polymere), wie sie in Patentschrift WO 98/58673 beschrieben wird, ist ebenfalls möglich, und kann genutzt werden, um die biologischen Eigenschaften der Partikel-Wirkstoff-Konjugate zu verbessern. Ebenso können weitere Moleküle angekoppelt werden, die dem Gesamtkonstrukt Zielfindungseigenschaften verleihen (z.B. polyklonale Antikörper, monoklonale Antikörper, humanifizierte Antikörper, humane Antikörper, chimere Antikörper, rekombinante Antikörper, bispezifische Antikörper, Antikörperfragmente, Aptamere, Fab-Fragmente, Fc-Fragmente, Peptide, Peptidomimetika, gap-Mere, Ribozymes, CpG-Oligomere, DNA-Zyme, Riboswitches, oder Lipide). Voraussetzung ist, dass alle weiteren Modifizierungen die aktivierbare Freisetzung der therapeutisch wirksamen Substanzen am Zielort nicht behindern.

Zudem ist es bevorzugt, die therapeutisch wirksame(n) Substanz(en) nicht direkt an ein Nanopartikel zu binden, sondern über ein Linker-Molekül zu immobilisieren. Als Linker können somit diverse Moleküle mit bis zu 50 Kohlenstoffatomen dienen, vorausgesetzt, der Linker enthält eine thermisch, photochemisch oder enzymatisch spaltbare Gruppe, säurelabile Gruppe oder sonst wie leicht ablösbare Gruppe. Eine Bindung im Linker-Molekül und/oder die Bindung des Linkers zum Wirkstoff und/oder die Bindung des Linkers zur Oberfläche des Nanopartikels müssen somit entweder durch die Einwirkung des magnetischen Wechselfeldes direkt oder indirekt spaltbar sein. Eine indirekte Spaltung ist dann gegeben, wenn beispielsweise durch das magnetische Wechselfeld Enzyme wie z.B. Peptidasen, Esterasen oder Hydrolasen am Zielort, z.B. in der Krebszelle angeregt oder deren Aktivität oder Expression gesteigert wird und diese Enzyme die vorgenannte Spaltung bewirken können. Zudem kann eine indirekte Spaltung bei der Verwendung von magnetischen Nanopartikel erfolgen, wenn diese durch das magnetische Wechselfeld erwärmt werden und dadurch eine thermisch labile Bindung gespalten wird. Denkbar ist auch die Erhöhung des pH-Werts am Zielort durch Einwirkung des magnetischen Wechselfeldes und die nachfolgende Spaltung von säurelabilen Bindungen im Linker-Molekül.

Als enzymatisch spaltbare Gruppe im oder am Linker-Molekül ist die Amidgruppe zu nennen. Thermisch oder mittels Säure spaltbare Gruppen umfassen z.B. Phosphatgruppen, Thiophosphatgruppen, Sulfatgruppen, Phosphamidgruppen, Carbamatgruppen oder Imingruppen.

Das Linker-Molekül kann ebenso ein Nukleinsäuremolekül, ein Polypeptid, eine Peptid-Nukleinsäure, ein Aptamer, DNA, RNA, ein Leucin-Zipper, ein Oligonukleotid, Biotin, Avidin, Streptavidin, eine Hapten-Antikörper-Brücke oder eine Biotin-Avidin-Brücke sein.

Der Wirkstoff muss nicht notwendigerweise kovalent an den Linker gebunden werden, sondern kann auch ionisch oder über Wasserstoffbrücken gebunden oder interkaliert oder komplexiert vorliegen.

Die diversen Möglichkeiten der Anbindung einer therapeutisch wirksamen Substanz wie beispielsweise von einem Antikrebsmittel, monoklonalen Antikörper, Aptamer, Nukleinsäure, Aminosäure, Peptid, Protein, Kohlenhydrat, Lipid, Glycoprotein, Glycan, Lipoprotein, oder von einem anti-proliferativen, anti-migrativen, anti-angiogenen, anti-thrombotischen, anti-inflammatorischen, antiphlogistischen, zytostatischen, zytotoxischen, anti-koagulativen, anti-bakteriellen, anti-viralen oder anti-mykotischen Wirkstoff an Mikropartikel und Nanopartikel sind eingehend in WO 2006108405 A beschrieben.

Somit kann das erfindungsgemäße Verfahren einen weiteren Schritt L1) umfassen, der die Anbindung von einem Antikrebsmittel, monoklonalen Antikörper, Aptamer, Nukleinsäure, Aminosäure, Peptid, Protein, Kohlenhydrat, Lipid, Glycoprotein, Glycan, Lipoprotein, oder von einem anti-proliferativen, anti-migrativen, anti-angiogenen, anti-thrombotischen, anti-inflammatorischen, antiphlogistischen, zytostatischen, zytotoxischen, anti-koagulativen, anti-bakteriellen, anti-viralen oder anti-mykotischen Wirkstoff an die gemäß Schritt K1) erhaltenen Partikel betrifft.

Ferner besteht auch die Möglichkeit, die Wirkstoffe adsorptiv an die Oberfläche der Nanopartikel zu binden und mit einer Barriereschicht zu überziehen, welche die Freisetzung des Wirkstoffs weitgehend verhindert bis die Barriereschicht durch Einwirkung eines magnetischen Wechselfeldes derart verändert insbesondere abgebaut wird, dass die Freisetzung des Wirkstoffs erfolgen kann.

### Figurenbeschreibung

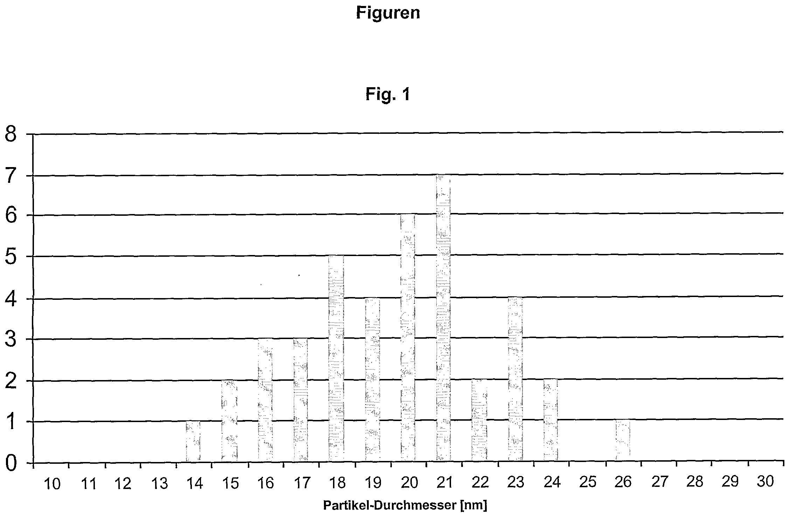

- Fig. 1:: zeigt die Partikelgrößenverteilung (hergeleitet aus Transmissions-Elektronenmikroskopie-Aufnahmen) von erfindungsgemäßen Eisenoxid-Nanopartikeln.

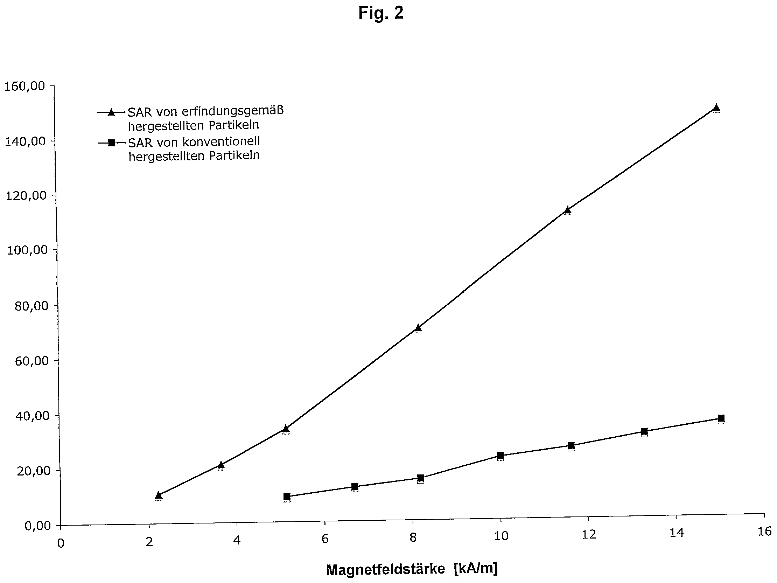

- Fig. 2:: zeigt SAR-Werte von erfindungsgemäßen Eisenoxid-Nanopartikeln in Wasser im Vergleich zum SAR-Wert herkömmlicher Eisenoxid-Nanopartikel, hergestellt durch eine Fällung gemäß Patentschrift DE 196 14 136 A1. Die SAR-Werte beziehen sich auf ein Magnetwechselfeld mit einer Frequenz von 100 kHz.

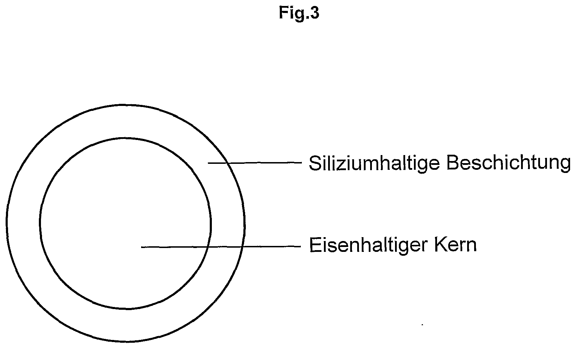

- Fig.3:: zeigt eine schematische Darstellung der erfindungsgemäßen eisenhaltigen Nanopartikel mit Kern und Hülle.

### Allgemeine Synthesevorschrift zur Herstellung der erfindungsgemäßen Partikel

### Schritt A1)

Zur Herstellung von Partikel-Keimen in einem organischen Lösungsmittel LM1 mit einem Siedepunkt von ca. 200°C bis ca. 400°C werden in einen Glaskolben 0,02 mol einer eisenhaltigen Verbindung A und 100 ml Lösungsmittel LM1 gegeben.

### Schritt A2)

Optional kann nun eines der hierin beschriebenen Additive in einer Menge von 0,008 bis 0,05 mol zugesetzt werden.

### Schritt B1)

Die Lösung wird mindestens 10 Minuten und vorzugsweise 1 Stunde auf eine Temperatur zwischen 50°C und 350°C erwärmt, welche ca. 50°C unter der nachfolgenden Reaktionstemperatur liegt.

### Schritt B2)

Optional kann nun weiteres Additiv als auch eine weitere eisenhaltigen Verbindung B zugesetzt werden.

### Schritt C1)

Das erhaltene Gemisch wird in einem Dreihalskolben mit Rückflusskühlung unter Schutzgas-Vorbeileitung auf die Siedetemperatur des jeweiligen Lösungsmittels LM1 bzw. LM2 erhitzt, welche mindestens 50°C höher ist, als die Temperatur der Heizphase gemäß Schritt B1) und für ca. 1 Stunde auf dieser Temperatur gehalten.

### Schritt C2)

Die erhaltenen Eisenoxid-Nanopartikel können nun optional oxidiert werden.

### Schritt D1)

Nun erfolgt die Reinigung der Partikel mittels Zentrifugation, Waschen und vorzugsweise mittels Soxhletextraktion.

### Schritt D1*)

Optional kann mindestens eine Temperphase der Eisenoxid-Nanopartikel durchgeführt werden.

### Schritt D2)

Sofern noch nicht geschehen, können die Eisenoxid-Nanopartikel nun optional oxidiert werden.

### Schritt D2*)

Optional kann mindestens eine Temperphase der Eisenoxid-Nanopartikel durchgeführt werden.

### Schritt E1)

Zur Dispergierung oder Suspendierung der gereinigten Partikel werden diese in Wasser mit neutralem pH-Wert oder in einer sauren wässrigen Lösung aufgenommen, welche vorzugsweise eine Mineralsäure enthält. Die Konzentration an Säure beträgt 0,002 bis 0,1 M. Um die Dispergierung oder Suspendierung zu unterstützen, kann eine Ultraschallbehandlung vorgenommen werden.

### Schritt E2)

Sofern noch nicht geschehen, können die Eisenoxid-Nanopartikel nun optional oxidiert werden.

### Schritt F1)

Nun erfolgt die Zugabe einer oberflächenaktiven Verbindung in einer Menge von 3 bis 8 mmol.

### Schritt F2)

Sofern noch nicht geschehen, können die Eisenoxid-Nanopartikel nun optional oxidiert werden.

### Schritt G1)

Optional wird danach vorzugsweise für 1 bis 2 Std. bei 50°C bis 90°C gerührt. Daran schließt sich für 1 bis 3 Std. unter Rühren die Behandlung mit Ultraschall an.

### Schritt G2)

Sofern noch nicht geschehen, können die Eisenoxid-Nanopartikel nun optional oxidiert werden.

### Schritt H1)

Nun erfolgt die Reinigung der erhaltenen Partikel mittels Zentrifugation, Waschen, Extraktion und / oder Dialyse, je nachdem, welches Verfahren oder Verfahrenskombination besser ist.

### Schritt H2)

Sofern noch nicht geschehen, können die Eisenoxid-Nanopartikel nun optional oxidiert werden.

### Schritt I1)

Die erhaltenen Partikel werden in einem Wasser-Alkohol-Gemisch (1:1 bis 5:1) aufgenommen, welches optional ein Amin und vorzugsweise Ammoniak in geringen Konzentrationen enthält.

### Schritt I2)

Sofern noch nicht geschehen, können die Eisenoxid-Nanopartikel nun optional oxidiert werden.

### Schritt J1)

Nun erfolgt die Zugabe eines Alkoxysilans in einer Menge von 0,04 - 0,08 mol.

### Schritt J2)

Sofern noch nicht geschehen, können die Eisenoxid-Nanopartikel nun optional oxidiert werden.

### Schritt K1)

Nun erfolgt die Reinigung der erhaltenen Partikel mittels Zentrifugation, Dialyse, Waschen und / oder Redispergierung in Wasser, je nachdem, welches Verfahren oder Verfahrenskombination besser ist.

### Schritt K2)

Sofern noch nicht geschehen, können die Eisenoxid-Nanopartikel nun optional oxidiert werden.

### Schritt L1)

Optional kann die Anbindung von Wirkstoffen an die Eisenoxid-Nanopartikel erfolgen.

### Beispiele

### Beispiel 1:

Zur Herstellung von Partikel-Keimen in Diethylenglykol-Dibutylether wurden in einem Glaskolben 0,3 g Eisenpentacarbonyl in 50 ml Diethylenglykol-Dibutylether gelöst. Zur Lösung wurden 1,7 g Ölsäure gegeben. Die Lösung wurde für 1 Stunde auf 150°C erhitzt.

### Beispiel 2:

Zur Herstellung von Partikel-Keimen in Polyglycol DME 500 (Firma Clariant) wurden in einem Glaskolben 8 g Eisen(III)oleat in 50 ml Polyglycol DME 500 gelöst. Zur Lösung wurden 1,5 g Ölsäure gegeben. Die Lösung wurde für 30 Minuten auf 120°C erhitzt.

### Beispiel 3A:

Zur Herstellung von Eisenoxid-Nanopartikeln wurden die Lösungen aus Beispiel 1-2 in einem Dreihalskolben mit Rückflusskühlung unter Schutzgas-Vorbeileitung (Argon) auf die Siedetemperatur des jeweiligen Lösungsmittels erhitzt und für 1 Stunde auf dieser Temperatur gehalten. Dabei färbte sich die Lösung schwarz. Die Partikel wurden nach dem Abkühlen durch Durchleiten von Luftsauerstoff über Nacht oxidiert.

### Beispiel 3B:

Zur Herstellung von Eisenoxid-Nanopartikeln wurden die Lösungen aus Beispiel 1-2 in einem Dreihalskolben mit Rückflusskühlung unter Schutzgas-Vorbeileitung (Argon) auf die Siedetemperatur des jeweiligen Lösungsmittels erhitzt und für 1 Stunde auf dieser Temperatur gehalten. Dabei färbte sich die Lösung schwarz.

### Beispiel 4:

Die Partikel aus Beispiel 3 wurden bei hohen g-Zahlen abzentrifugiert und mit Ethanol gewaschen. 500 mg des gewaschenen Produktes wurden in eine Extraktionshülse (603g Firma Whatman) eingewogen und in eine Soxhletapparatur eingesetzt. 200 ml Ethanol als Extraktionsmittel wurden in den Vorlagekolben der Soxhletapparatur eingefüllt. Das Extraktionsmittel wurde zum Sieden erhitzt. Die kontinuierliche Extraktion wurde über 8 h durchgeführt und beinhaltete ca. 16 Extraktionszyklen. Dabei verfärbt sich die Ethanollösung gelblich. Nach Beendigung wurde die Extraktionshülse entnommen und das Pulver in ein Schlenkgefäß überführt und 1 h im Vakuum getrocknet.

### Beispiel 5:

Zur Dispergierung der Partikel nach der Extraktion wurden 0,5 g des Nanopartikel-Pulvers aus Beispiel 4 in 20 ml 0,01 M HCl suspendiert. Dann wurden die Nanopartikel für 30 Minuten mit Ultraschall behandelt. Danach wurde 0,5 g festes Natriumoleat zugegeben.

### Schritt G1:

Danach wurde für 1,5 Std. bei 70°C gerührt und anschließend 2 Std. unter Rühren mit Ultraschall behandelt. Nach erfolgreicher Dispergierung wurde die Dispersion bei niedrigen g-Zahlen zentrifugiert, um nicht-dispergierte Partikel abzutrennen. Danach wurde die verbleibende Dispersion gewaschen, um überschüssiges Natriumoleat zu entfernen. Dies erfolgt durch Zentrifugation bei hohen g-Zahlen und Waschen mit Diethylether und Redispergierung in Wasser. Alternativ kann eine Extraktion mit Diethylether oder eine Dialyse erfolgen. Zur vollständigen Redispergierung wurde die Dispersion mit Ultraschall behandelt.

### Beispiel 6:

Zu 120 ml einer Mischung aus Wasser / Ethanol (3:1) und 1,5 Gew.% Ammoniak wurden 3,3 ml einer Partikel-Dispersion gemäß Beispiel 5 (0,97 mol/l Fe) und 2,14 ml Tetraethoxysilan gegeben. Die Dispersion wurde während der Zugabe gerührt und danach für 6 Stunden mit Ultraschall behandelt. Die Dispersion wurde durch Zentrifugation und Redispergierung in Wasser aufgereinigt.

### Beispiel 7 (Temperphase):

Die in Beispiel 4 erhaltenen Partikel wurden in 200 ml Diethylenglykol-dibutylether suspendiert. Dann wurde für 12 Stunden bei 80°C mit Luft begast und anschließend für 8 Stunden unter Rückfluss gekocht (Sdp. ca. 256°C). Die Suspension wurde danach langsam (innerhalb von 8 Stunden) auf Raumtemperatur abgekühlt. Dieser Vorgang wurde 2 mal wiederholt.

Die so erhaltenen (getemperten) Partikel wurden gewaschen und in 20 ml 1M HNO₃ suspendiert. Dann wurden 0,3 mmol Eisennitrat (Fe(NO₃)₃ * 9 H₂O) zugegeben und für eine Stunde unter Rückfluss gekocht (100°C). Die Partikel wurden 3 x mit je 100 ml Wasser gewaschen.

Diese Partikel wurden dann analog zu den Beispielen 4-6 beschichtet.

### Beispiel 8A (mit Oxidation / ohne Luftbegasung):

Zur Herstellung von Eisenoxid-Nanopartikeln in Ethylenglykol wurden 0,1 mol FeCl₃*6H₂O und 0,2 mol FeCl₃ (wasserfrei), 50 g Natriumacetat und 195 g Diaminohexan in 900 ml Ethylenglykol gelöst und für 1 Stunde auf 60°C erhitzt.
Dann wurde die Lösung innerhalb von 30 Minuten zum Siedepunkt erhitzt. Die Siedetemperatur wurde für 6 Stunden gehalten. Die entstandene Dispersion wurde langsam auf Raumtemperatur abgekühlt.
Die Partikel wurden 3mal mit einer Mischung aus Ethanol/Wasser gewaschen. Danach wurden die Partikel erneut in 900 ml Ethylenglykol suspendiert. Die Suspension wurde zum Siedepunkt des Ethylenglykols erhitzt und für 24 Stunden auf dieser Temperatur gehalten.
Nach dem Abkühlen wurden die Partikel mit Wasser/Ethanol gewaschen und in 900 ml 1 M HNO₃ suspendiert. Dann wurden 450 ml einer 0,7M Eisennitrat-Lösung (Fe(NO₃)₃ * 9 H₂O) zugegeben und für eine Stunde unter Rückfluss gekocht (100°C). Die Partikel wurden 3 x mit je 500 ml Wasser gewaschen.

Diese Partikel wurden analog zu den Beispielen 4-6 beschichtet.

### Beispiel 8B (ohne Oxidation / mit Luftbegasung):

Zur Herstellung von Eisenoxid-Nanopartikeln in Ethylenglykol wurden 0,1 mol FeCl₃*6H₂O und 0,2 mol FeCl₃ (wasserfrei), 50 g Natriumacetat und 195 g Diaminohexan in 900 ml Ethylenglykol gelöst und für 1 Stunde auf 60°C erhitzt.
Dann wurde die Lösung innerhalb von 30 Minuten zum Siedepunkt erhitzt. Die Siedetemperatur wurde für 6 Stunden gehalten. Die entstandene Dispersion wurde langsam auf Raumtemperatur abgekühlt.

Die Partikel wurden 3mal mit einer Mischung aus Ethanol/Wasser gewaschen. Danach wurden die Partikel erneut in 900 ml Ethylenglykol suspendiert und mit Luftsauerstoff begast. Die Suspension wurde zum Siedepunkt des Ethylenglykols erhitzt und für 24 Stunden auf dieser Temperatur gehalten.
Nach dem Abkühlen wurden die Partikel mit Wasser/Ethanol gewaschen und in Wasser suspendiert.

Diese Partikel wurden analog zu den Beispielen 4-6 beschichtet

### Beispiel 8C (mit Oxidation / mit Luftbegasung):

Zur Herstellung von Eisenoxid-Nanopartikeln in Ethylenglykol wurden 0,1 mol FeCl₃*6H₂O und 0,2 mol FeCl₃ (wasserfrei), 50 g Natriumacetat und 195 g Diaminohexan in 900 ml Ethylenglykol gelöst und für 1 Stunde auf 60°C erhitzt.
Dann wurde die Lösung innerhalb von 30 Minuten zum Siedepunkt erhitzt. Die Siedetemperatur wurde für 6 Stunden gehalten. Die entstandene Dispersion wurde langsam auf Raumtemperatur abgekühlt.
Die Partikel wurden 3mal mit einer Mischung aus Ethanol/Wasser gewaschen. Danach wurden die Partikel erneut in 900 ml Ethylenglykol suspendiert und mit Luftsauerstoff begast. Die Suspension wurde zum Siedepunkt des Ethylenglykols erhitzt und für 24 Stunden auf dieser Temperatur gehalten.
Nach dem Abkühlen wurden die Partikel mit Wasser/Ethanol gewaschen und in 900 ml 1 M HNO₃ suspendiert. Dann wurden 450 ml einer 0,7M Eisennitrat-Lösung (Fe(NO₃)₃ * 9 H₂O) zugegeben und für eine Stunde unter Rückfluss gekocht (100°C). Die Partikel wurden 3 x mit je 500 ml Wasser gewaschen.

Diese Partikel wurden dann analog zu den Beispielen 4-6 beschichtet.

### Beispiel 8D (ohne Oxidation / ohne Luftbegasung):

Zur Herstellung von Eisenoxid-Nanopartikeln in Ethylenglykol wurden 0,1 mol FeCl₃*6H₂O und 0,2 mol FeCl₃ (wasserfrei), 50 g Natriumacetat und 195 g Diaminohexan in 900 ml Ethylenglykol gelöst und für 1 Stunde auf 60°C erhitzt.
Dann wurde die Lösung innerhalb von 30 Minuten zum Siedepunkt erhitzt. Die Siedetemperatur wurde für 6 Stunden gehalten. Die entstandene Dispersion wurde langsam auf Raumtemperatur abgekühlt.
Die Partikel wurden 3mal mit einer Mischung aus Ethanol/Wasser gewaschen. Danach wurden die Partikel erneut in 900 ml Ethylenglykol suspendiert. Die Suspension wurde zum Siedepunkt des Ethylenglykols erhitzt und für 24 Stunden auf dieser Temperatur gehalten.

Nach dem Abkühlen wurden die Partikel mit Wasser/Ethanol gewaschen und in Wasser suspendiert.

Diese Partikel wurden analog zu den Beispielen 4-6 beschichtet

### Beispiel 9

Zur Herstellung von Eisenoxid-Nanopartikeln wurde eine Lösung von 96 g Natriumhydroxid und 680 ml Ölsäure in 2000 ml Methanol zu einer Lösung aus 216 g Eisen(III)Chlorid-Hexahydrat in 500 ml Methanol gegeben. Der entstehende Feststoff wurde mit Methanol gewaschen und in Diethylether gelöst. Dann wurde mehrmals mit Wasser extrahiert. Der Feststoff wurde mit Aceton ausgefällt, gewaschen und im Vakuum getrocknet.

75 g dieses Feststoffs wurden in 250 ml Trioctylamin gelöst und für 1 Stunde auf 120°C erhitzt.

Dann wurde die Lösung in einem Autoklaven innerhalb von 30 Minuten auf 380°C erhitzt. Diese Temperatur wurde für 4 Stunden gehalten. Die entstandene Dispersion wurde langsam auf Raumtemperatur abgekühlt.
Die Partikel wurden 3mal mit einer Mischung aus Ethanol/Wasser gewaschen.

Danach wurden die Partikel in 300 ml Diethylenglykol-dibutylether suspendiert und mit Luftsauerstoff begast. Die Suspension wurde im Autoklaven auf 300°C erhitzt für 24 Stunden auf dieser Temperatur gehalten.

Diese Partikel wurden wie in Beispiel 8C oxidiert und dann analog zu den Beispielen 4-6 beschichtet.

## Patentansprüche

1. Verfahren zur Herstellung von Partikeln, wobei die Partikel einen SAR (Spezifische Absorptionsrate)-Wert von 10 - 40 W pro g Fe bei einer Magnetfeldstärke von 4 kA/m und einer Frequenz eines magnetischen Wechselfelds von 100 kHz aufweisen, umfassend die folgenden Schritte:
A1) Bereitstellung einer Zusammensetzung mindestens einer eisenhaltigen Verbindung A in mindestens einem organischen Lösungsmittel LM1,
B1) Erhitzen der Zusammensetzung für mindestens 10 Minuten auf eine Temperatur im Bereich von 50 °C bis zu einer Temperatur von 50 °C unterhalb der eigentlichen Reaktionstemperatur gemäß Schritt C1 der eisenhaltigen Verbindung A,
C1) Erhitzen der Zusammensetzung auf eine Temperatur zwischen 200 °C und 400 °C, wobei die Dauer der Heizphase mindestens 30 Minuten beträgt,
D1) Reinigung der erhaltenen Partikel mittels Soxhlet-Extraktion, wobei die Dauer der Extraktion zwischen 1 und 8 Stunden liegt,
E1) Suspendierung der gereinigten Nanopartikel in Wasser oder einer wässrigen Lösung einer Säure,
F1) Zugabe einer oberflächenaktiven Verbindung zur wässrigen Suspension erhalten gemäß Schritt E1,
H1) Reinigung der erhaltenen wässrigen Dispersion der Partikel,
I1) Herstellung einer Dispersion der Partikel gemäß Schritt H1) in einem Lösungsmittelgemisch aus Wasser und einem mit Wasser mischbaren Lösungsmittel,
J1) Zugabe eines Alkoxysilans zur Dispersion der Partikel in dem Lösungsmittelgemisch gemäß Schritt I1),
K1) Reinigung der Partikel.

2. Verfahren nach Anspruch 1, des Weiteren umfassend den auf Schritt A1 folgenden Schritt A2):
A2) Zugabe eines Additivs ausgewählt aus der Gruppe umfassend:
Tenside, Silane, Si- oder Al-organische Verbindungen Phosphine, gesättigte oder ungesättigte Fettsäuren, Amine, Diamine, Carbonsäuren und deren Salze, gesättigte und ungesättigte Fettsäuren, Polymere.

3. Verfahren nach Anspruch 1 oder 2, des Weiteren umfassend den auf Schritt B1 folgenden Schritt B2):
B2) Zugabe eines Additivs ausgewählt aus der Gruppe umfassend:
Tenside, Silane, Si-oder AI-organische Verbindungen, Phosphine, gesättigte oder ungesättigte Fettsäuren, Amine, Diamine, Carbonsäuren und deren Salze, gesättigte und ungesättigte Fettsäuren, Polymere oder
B2) Zugabe einer Zusammensetzung mindestens einer eisenhaltigen Verbindung B in mindestens einem organischen Lösungsmittel L2 oder
B2) Zugabe einer Zusammensetzung mindestens einer eisenhaltigen Verbindung B in mindestens einem organischen Lösungsmittel L2 und Zugabe eines Additivs ausgewählt aus der Gruppe umfassend:
Tenside, Silane, Si- oder Al-organische Verbindungen, Phosphine, gesättigte oder ungesättigte Fettsäuren, Amine, Diamine, Carbonsäuren und deren Salze, gesättigte und ungesättigte Fettsäuren, Polymere.

4. Verfahren nach Anspruch 3, wobei die mindestens eine eisenhaltige Verbindung B aus der Gruppe ausgewählt wird umfassend Eisen-Komplexverbindungen, Eisencarbonyl-Verbindungen, Eisen-Salze, organische Eisenverbindungen, Eisensalze von gesättigten/ungesättigten Fettsäuren und Eisen-Sandwich-Komplexe.

5. Verfahren nach Anspruch 3, wobei das mindestens eine Lösungsmittel L2 ausgewählt wird aus der Gruppe umfassend hochsiedende Amine, Alkane, Olefine, Alkohole oder Ether, Alkylenglykolmonoether, Alkylenglykoldiether, Ethylenglykolmonoether, Ethylenglykoldiether, Propylenglykolmonoether, Propylenglykoldiether, Glycerinmonoether, Glycerindiether, Glycerintriether, Glykol-Diether (Glymes).

6. Verfahren nach Anspruch 3, wobei die mindestens eine eisenhaltige Verbindung B identisch zur mindestens einen eisenhaltigen Verbindung A ist und/oder das mindestens eine organische Lösungsmittel LM1 identisch zum mindestens einen organischen Lösungsmittel LM2 ist.

7. Verfahren nach einem der vorherigen Ansprüche, des Weiteren umfassend den auf Schritt C1 oder D1 oder E1 oder F1 oder G1 oder H1 oder I1 oder J1 oder K1 folgende Schritt X2):
X2) Oxidation der gebildeten Partikel.

8. Verfahren nach einem der vorherigen Ansprüche, des Weiteren umfassend nach Schritt F1 den Schritt G1):
G1) Behandlung der wässrigen Suspension gemäß Schritt F1) mit Ultraschall.

9. Verfahren nach einem der vorherigen Ansprüche, des Weiteren umfassend den auf Schritt D1 folgenden Temperschritt D1*) oder den auf D2 folgenden Temperschritt D2*):
D1*) Tempern der erhaltenen Partikel oder
D2*) Tempern der erhaltenen Partikel.

10. Verfahren nach einem der vorherigen Ansprüche, wobei die mindestens eine eisenhaltige Verbindung A aus der Gruppe ausgewählt wird umfassend Eisen-Kompiexverbindungen, Eisencarbonyl-Verbindungen, Eisen-Salze, organische Eisenverbindungen, Eisensalze von gesättigten/ungesättigten Fettsäuren und Eisen-Sandwich-Komplexe.

11. Verfahren nach einem der vorherigen Ansprüche, wobei das mindestens eine Lösungsmittel LM1 ausgewählt wird aus der Gruppe umfassend hochsiedende Amine, Alkane, Olefine, Alkohole, Ether, Alkylenglykolmonoether, Alkylenglykoldiether, Ethylenglykolmonoether, Ethylenglykoldiether, Propylenglykolmonoether, Propylenglykoldiether, Glycerinmonoether, Glycerindiether, Glycerintriether, Glykol-Diether (Glymes).

12. Verfahren nach einem der vorherigen Ansprüche, wobei im Reinigungsschritt gemäß Schritt D1) die eventuell vorhandenen Additive weitgehend entfernt werden.

13. Verfahren nach einem der vorherigen Ansprüche, wobei die oberflächenaktive Verbindung gemäß Schritt F1) ausgewählt wird aus der Gruppe umfassend: Fettsäuren, Fettsäuresalze, Tenside, Polymere, Polyvinylalkohol, Polyethylenglykol, Polyacrylsäure, Dextran, PLGA, Chitosan und Polyethylenimin.

14. Verfahren nach einem der vorherigen Ansprüche, des Weiteren umfassend der Schritt L1):
L1) Anbindung von einem Antikrebsmittel, monoklonalen Antikörper, Aptamer, Nukleinsäure, Aminosäure, Peptid, Protein, Kohlenhydrat, Lipid, Glycoprotein, Glycan, Lipoprotein, oder von einem anti-proliferativen, anti-migrativen, anti-angiogenen, anti-thrombotischen, antiinflammatorischen, antiphlogistischen, zytostatischen, zytotoxischen, anti-koagulativen, anti-bakteriellen, anti-viralen oder antimykotischen Wirkstoff an die gemäß Schritt K1) erhaltenen Partikel.

15. Verfahren nach einem der vorherigen Ansprüche, wobei die in Schritt B1) erreichte Temperatur bevorzugt für mindestens 30 Minuten und insbesondere bevorzugt für 40 Minuten gehalten wird.

16. Verfahren nach einem der vorherigen Ansprüche, wobei die Dauer der Heizphase in Schritt C1) bevorzugt 1 - 30 Stunden, weiter bevorzugt 10 - 20 Stunden und insbesondere bevorzugt 15 Stunden beträgt.

17. Verfahren nach einem der vorherigen Ansprüche, wobei die Dispersion in Schritt J1) für 1 bis 8 Stunden, bevorzugt für 3 bis 5 Stunden und insbesondere bevorzugt für 4 Stunden mit Ultraschall behandelt wird.

18. Eisenhaltige Partikel, wobei die Partikel einen SAR (Spezifische Absorptionsrate)-Wert von 10 - 40 W pro g Fe bei einer Magnetfeldstärke von 4 kA/m und einer Frequenz eines magnetischen Wechselfelds von 100 kHz haben und eine siliziumhaltige Hülle aufweisen.

19. Eisenhaltige Partikel nach Anspruch 18, wobei die siliziumhaltige Hülle eine Dicke zwischen 0,5 und 10 nm, bevorzugt zwischen 1 bis 6 nm, weiter bevorzugt zwischen 2 bis 4 nm und insbesondere von 3 nm hat.

20. Eisenhaltige Partikel nach Anspruch 18 oder 19, wobei die Partikel ferromagnetisch, ferrimagnetisch oder superparamagnetisch sind.

21. Eisenhaltige Partikel nach einem der Ansprüche 18 bis 20, wobei die Partikel einen SAR-Wert zwischen 20 - 40 W pro g Fe, bevorzugt zwischen 25 bis 40 W pro g Fe, insbesondere zwischen 30 - 40 W pro g Fe bei einer Magnetfeldstärke von 4 kA/m und einer Frequenz eines magnetischen Wechselfelds von 100 kHz haben.

22. Eisenhaltige Partikel nach einem der Ansprüche 18 bis 21, wobei die Partikel einen Durchmesser von weniger als 500 nm, bevorzugt von 1 bis 100 nm, insbesondere bevorzugt von 15 bis 30 nm aufweisen.

23. Eisenhaltige Partikel nach einem der Ansprüche 18 bis 22, wobei die siliziumhaltige Hülle durch Alkoxysilane funktionalisiert ist.

24. Eisenhaltige Partikel nach Anspruch 23, wobei die Alkoxysilane bevorzugt Trialkoxysilane sind, die
(i) eine über eine Si-C-Bindung gebundene funktionelle Gruppe tragen, bevorzugt (3-acryloxypropyl)Trimethoxysilan, Triethoxysilylbutyraldehyd, 3-Aminopropyltrlethoxysilan, 3-Isocyanatopropyltriethoxysilan, oder
(ii) eine über Si-C gebundene Polyethylenglykol-Seitenkette unterschiedlicher Länge tragen, bevorzugt 2-[methoxy(Polyethylenoxy)Propyl]Trimethoxysilan.

25. Pharmazeutische Zusammensetzung enthaltend eisenhaltige Partikeln gemäß einem der Ansprüche 18 bis 24.

## Claims

1. A method for the production of particles, wherein the particles have a SAR (specific absorption rate) value of 10 - 40 W per g Fe at a magnetic field strength of 4 kA/m and a frequency of an alternating magnetic field of 100 kHz, comprising the following steps:
A1) providing a composition of at least one iron-containing compound A in at least one organic solvent LM1,
B1) heating of the composition to a temperature in the range of 50 °C to a temperature of 50 °C below the actual reaction temperature according to step C1 of the iron-containing compound A for at least 10 minutes,
C1) heating of the composition to a temperature of between 200 °C and 400 °C, wherein the duration of the heating phase is at least 30 minutes,
D1) purification of the obtained particles by means of Soxhlet extraction, wherein the duration of the extraction is between 1 and 8 hours,
E1) suspending the purified nanoparticles in water or an aqueous solution of an acid,
F1) addition of a surface-active compound to the aqueous suspension obtained according to step E1,
H1) purification of the obtained aqueous dispersion of the particles,
I1) production of a dispersion of the particles according to step H1) in a solvent mixture of water and a solvent miscible with water,
J1) addition of an alkoxysilane to the dispersion of the particles in the solvent mixture according to step I1),
K1) purification of the particles.

2. Method according to claim 1, further comprising the step A2) following step A1:
A2) addition of an additive selected from the group comprising:
tensides, silanes, Si- or Al-organic compounds, phosphines, saturated or unsaturated fatty acids, amines, diamines, carboxylic acids and salts thereof, saturated and unsaturated fatty acids, polymers.

3. Method according to claim 1 or 2, further comprising step B2) following step B1:
B2) addition of an additive selected from the group comprising:
tensides, silanes, Si- or Al-organic compounds, phosphines, saturated or unsaturated fatty acids, amines, diamines, carboxylic acids and salts thereof, saturated and unsaturated fatty acids, polymers, or
B2) addition of a composition of at least one iron-containing compound B in at least one organic solvent L2, or
B2) addition of a composition of at least one iron-containing compound B in at least one organic solvent L2, and addition of an additive selected from the group comprising: tensides, silanes, Si- or Al-organic compounds, phosphines, saturated or unsaturated fatty acids, amines, diamines, carboxylic acids and salts thereof, saturated and unsaturated fatty acids, polymers.

4. Method according to claim 3, wherein the at least one iron-containing compound B is selected from the group comprising iron complex compounds, iron carbonyl compounds, iron salts, organic iron compounds, iron salts of saturated/unsaturated fatty acids, and iron-sandwich complexes.

5. Method according to claim 3, wherein the at least one solvent L2 is selected from the group comprising high-boiling amines, alkanes, olefins, alcohols or ethers, alkylene glycol monoethers, alkylene glycol diethers, ethylene glycol monoethers, ethylene glycol diethers, propylene glycol monoethers, propylene glycol diethers, glycerin monoethers, glycerin diethers, glycerin triethers, glycol diethers (glymes).

6. Method according to claim 3, wherein the at least one iron-containing compound B is identical to the at least one iron-containing compound A and/or the at least one organic solvent LM1 is identical to the at least one organic solvent LM2.

7. Method according to one of the preceding claims, further comprising the step X2) following steps C1 or D1 or E1 or F1 or G1 or H1 or I1 or J1 or K1:
X2) oxidation of the formed particles.

8. Method according to one of the preceding claims, further comprising following step F1 the step G1):
G1) treatment of the aqueous suspension according to step F1) with ultrasound.

9. Method according to one of the preceding claims, further comprising the tempering step D1*) following step D1 or the tempering step D2*) following D2:
D1*) tempering of the obtained particles, or
D2*) tempering of the obtained particles.

10. Method according to one of the preceding claims, wherein the at least one iron-containing compound A is selected from the group comprising
iron complex compounds, iron carbonyl compounds, iron salts, organic iron compounds, iron salts of saturated/unsaturated fatty acids and iron-sandwich complexes.

11. Method according to one of the preceding claims, wherein the at least one solvent LM1 is selected from the group comprising
high-boiling amines, alkanes, olefins, alcohols, ethers, alkylene glycol monoethers, alkylene glycol diethers, ethylene glycol monoethers, ethylene glycol diethers, propylene glycol monoethers, propylene glycol dietners, glycerin monoethers, glycerin diethers, glycerin triethers, glycol diethers (glymes).

12. Method according to one of the preceding claims, wherein in the purification step according to step D1) the potentially present additives are substantially removed.

13. Method according to one of the preceding claims, wherein the surface-active compound according to step F1) is selected from the group comprising: fatty acids, fatty acid salts, tensides, polymers, polyvinyl alcohol, polyethylene glycol, polyacrylic acid, dextran, PLGA, chitosan and polyethylene imine.

14. Method according to one of the preceding claims, further comprising the step L1):
L1) Coupling of an anticancer compound, a monoclonal antibody, an aptamer, a nucleic acid, an amino acid, a peptide, a protein, a carbohydrate, a lipid, a glycoprotein, a glycan, a lipoprotein, or of an anti-proliferative, anti-migrative, anti-angiogenic, anti-thrombotic, anti-inflammatory, anti-phlogistic, cytostatic, cytotoxic, anti-coagulative, anti-bacterial, anti-viral, or anti-mycotic drug to the particles obtained according to step K1).

15. Method according to one of the preceding claims, wherein the temperature obtained in step B1) is preferably retained for at least 30 minutes and particularly preferably for 40 minutes.

16. Method according to one of the preceding claims, wherein the duration of the heating phase in step C1) is preferably 1 - 30 hours, more preferably 10 - 20 hours, and particularly preferably 15 hours.

17. Method according to one of the preceding claims, wherein the dispersion in step J1) is treated with ultrasound for 1 to 8 hours, preferably for 3 to 5 hours, and particularly preferably for 4 hours.

18. Iron-containing particles, wherein the particles nave a SAR (specific absorption rate) value of 10 - 40 W per g Fe at a magnetic field strength of 4 kA/m and a frequency of an alternating magnetic field of 100 kHz and have a silicon-containing shell.

19. Iron-containing particles according to claim 18, wherein the silicon-containing shell has a thickness of between 0.5 and 10 nm, preferably between 1 to 6 nm, more preferably between 2 to 4 nm, and particularly of 3 nm.

20. Iron-containing particles according to claim 18 or 19, wherein the particles are ferromagnetic, ferrimagnetic or superparamagnetic.

21. Iron-containing particles according to one of claims 18 to 20, wherein the particles have a SAR value of between 20 - 40 W per g Fe, preferably between 25 to 40 W per g Fe, particularly between 30 - 40 W per g Fe at a magnetic field strength of 4 kA/m and a frequency of an alternating magnetic field of 100 kHz.

22. Iron-containing particles according to one of claims 18 to 21, wherein the particles have a diameter of less than 500 nm, preferably of 1 to 100 nm, particularly preferably of 15 to 30 nm.

23. Iron-containing particles according to one of claims 18 to 22, wherein the silicon-containing shell is functionalized by alkoxysilanes.

24. Iron-containing particles according to claim 23, wherein the alkoxysilanes are preferably trialkoxysilanes, which
(i) bear a functional group coupled by an Si-C bond, preferably (3-acryloxypropyl)trimethoxysilane, triethoxysilyl-butyraldehyde, 3-aminopropyltriethoxysilane, or 3-isocyanato-propyltriethoxysilane, or
(ii) bear a polyethylene glycol side chain of different length coupled by Si-C, preferably 2-[methoxy(polyethyleneoxy)propyl]-trimethoxysilane.

25. Pharmaceutical composition containing iron-containing particles according to one of claims 18 to 24.

## Revendications

1. Procédé de production de particules, dans lequel les particules présentent une valeur SAR (taux d'absorption spécifique) de 10 à 40 W par g de Fe à une force de champ magnétique de 4 kA/m et à une fréquence d'un champ alternatif magnétique de 100 kHz, comportant les étapes suivantes :
A1) mise à disposition d'une composition d'au moins un composé contenant du fer A dans au moins un solvant organique LM1,
B1) chauffage de la composition pendant au moins 10 minutes à une température située dans la plage de 50°C jusqu'à une température de 50°C en dessous de la température de réaction proprement dite selon l'étape C1 du composé contenant du fer A,
C1) chauffage de la composition à une température située entre 200°C et 400°C, dans lequel la durée de la phase de chauffage est d'au moins 30 minutes,
D1) purification des particules obtenues au moyen de l'extraction de Soxhlet, dans laquelle la durée de l'extraction est située entre 1 et 8 heures,
E1) mise en suspension des nanoparticules purifiées dans de l'eau ou dans une solution aqueuse d'un acide,
F1) addition d'un composé tensioactif à la suspension aqueuse obtenue selon l'étape E1,
H1) purification de la dispersion aqueuse de particules obtenue,
I1) préparation d'une dispersion de particules selon l'étape H1) dans un mélange de solvants constitué d'eau et d'un solvant miscible avec l'eau,
J1) addition d'un alkoxysilane à la dispersion de particules dans le mélange de solvants selon l'étape I1),
K1) purification des particules.

2. Procédé selon la revendication 1, comprenant en outre l'étape A2) suivant l'étape A1 :
A2) addition d'un additif choisi dans le groupe constitué de :
tensioactifs, silanes, composés organiques Si ou Al, phosphines, acides gras saturés ou insaturés, amines, diamines, acides carboxyliques et leurs sels, acides gras saturés et insaturés, polymères.

3. Procédé selon la revendication 1 ou 2, comprenant en outre l'étape B2) suivant l'étape B1 :
B2) addition d'un additif choisi dans le groupe constitué de :
tensioactifs, silanes, composés organiques Si ou Al, phosphines, acides gras saturés ou insaturés, amines, diamines, acides carboxyliques et leurs sels, acides gras saturés et insaturés, polymères, ou
B2) addition d'une composition d'au moins un composé contenant du fer B dans au moins un solvant organique L2, ou
B2) addition d'une composition d'au moins un composé contenant du fer B dans au moins un solvant organique L2 et addition d'un additif choisi dans le groupe constitué de :
tensioactifs, silanes, composés organiques Si ou Al, phosphines, acides gras saturés ou insaturés, amines, diamines, acides carboxyliques et leurs sels, acides gras saturés et insaturés, polymères.

4. Procédé selon la revendication 3, dans lequel l'au moins un composé contenant du fer B est choisi dans le groupe comprenant les composés de complexes de fer, les composés de carbonyle ferreux, les sels de fer, les composés de fer organiques, les sels de fer d'acides gras saturés/insaturés et les complexes sandwichs de fer.

5. Procédé selon la revendication 3, dans lequel l'au moins un solvant L2 est choisi dans le groupe comprenant les amines à point d'ébullition élevé, les alcanes, les oléfines, les alcools ou éthers, les monoéthers d'alkylène-glycol, les diéthers d'alkylèneglycol, les monoéthers d'éthylèneglycol, les diéthers d'éthylèneglycol, les monoéthers de propylèneglycol, les diéthers de propylène-glycol, les monoéthers de glycérine, les diéthers de glycérine, les triéthers de glycérine, les diéthers de glycol (glymes).

6. Procédé selon la revendication 3, dans lequel l'au moins un composé contenant du fer B est identique à au moins un composé contenant du fer A et/ou l'au moins un solvant organique LM1 est identique à au moins un solvant organique LM2.

7. Procédé selon l'une des revendications précédentes, comprenant en outre l'étape suivante X2) suivant l'étape C1 ou D1 ou E1 ou F1 ou G1 ou H1 ou I1 ou J1 ou K1 :
X2) oxydation des particules formées.

8. Procédé selon l'une des revendications précédentes, comprenant en outre l'étape G1) après l'étape F1 :
G1) traitement de la suspension aqueuse selon l'étape F1) avec des ultrasons.

9. Procédé selon l'une des revendications précédentes, comprenant en outre l'étape de revenu D1*) suivant l'étape D1 ou l'étape de revenu D2*) suivant l'étape D2 :
D1*) revenu des particules obtenues ou
D2*) revenu des particules obtenues.

10. Procédé selon l'une des revendications précédentes, dans lequel l'au moins un composé contenant du fer A est choisi dans le groupe comprenant les composés de complexes de fer, les composés de carbonyle ferreux, les sels de fer, les composés de fer organiques, les sels de fer d'acides gras saturés/insaturés et les complexes sandwichs de fer.

11. Procédé selon l'une des revendications précédentes, dans lequel l'au moins un solvant LM1 est choisi dans le groupe comprenant les amines à point d'ébullition élevé, les alcanes, les oléfines, les alcools, les éthers, les monoéthers d'alkylèneglycol, les diéthers d'alkylèneglycol, les monoéthers d'éthylèneglycol, les diéthers d'éthylène-glycol, les monoéthers de propylèneglycol, les diéthers de propylèneglycol, les monoéthers de glycérine, les diéthers de glycérine, les triéthers de glycérine, les diéthers de glycol (glymes).

12. Procédé selon l'une des revendications précédentes, dans lequel les additifs éventuellement présents sont largement éliminés au cours de l'étape de purification selon l'étape D1).

13. Procédé selon l'une des revendications précédentes, dans lequel le composé tensioactif selon l'étape F1) est choisi dans le groupe comprenant : les acides gras, les sels d'acides gras, les tensioactifs, les polymères, l'alcool polyvinylique, le polyéthylèneglycol, l'acide polyacrylique, le dextrane, le PLGA, le chitosane et la polyéthylène-imine.

14. Procédé selon l'une des revendications précédentes, comprenant en outre l'étape L1) :
L1) la liaison d'un agent anticancéreux, d'un anticorps monoclonal, d'un aptamère, d'un acide nucléique, d'un acide aminé, d'un peptide, d'une protéine, d'un glucide, d'un lipide, d'une glycoprotéine, d'un glycane, d'une lipoprotéine, ou d'une substance active anti-proliférative, anti-migratoire, anti-angiogène, anti-thrombotique, anti-inflammatoire, anti-phlogistique, cytostatique, cytotoxique, anticoagulante, antibactérienne, antivirale ou antimycotique aux particules obtenues selon l'étape K1).

15. Procédé selon l'une des revendications précédentes, dans lequel la température atteinte à l'étape B1) est maintenue de préférence pendant au moins 30 minutes et particulièrement de préférence pendant 40 minutes.

16. Procédé selon l'une des revendications précédentes, dans lequel la durée de la phase de chauffage à l'étape C1) est de préférence de 1 à 30 heures, plus préférentiellement de 10 à 20 heures et particulièrement de préférence de 15 heures.

17. Procédé selon l'une des revendications précédentes, dans lequel la dispersion à l'étape J1) est traitée avec des ultrasons pendant 1 à 8 heures, de préférence pendant 3 à 5 heures et particulièrement de préférence pendant 4 heures.

18. Particules contenant du fer, lesdites particules présentant une valeur SAR (taux d'absorption spécifique) de 10 à 40 W par g de Fe à une force de champ magnétique de 4 kA/m et à une fréquence d'un champ alternatif magnétique de 100 kHz et ayant une enveloppe contenant du silicium.

19. Particules contenant du fer selon la revendication 18, dans lesquelles l'enveloppe contenant du silicium a une épaisseur située entre 0,5 et 10 nm, de préférence entre 1 et 6 nm, plus préférentiellement entre 2 et 4 nm et particulièrement de 3 nm.

20. Particules contenant du fer selon la revendication 18 ou 19, lesdites particules étant ferromagnétiques, ferrimagnétiques ou superparamagnétiques.

21. Particules contenant du fer selon l'une des revendications 18 à 20, lesdites particules ayant une valeur SAR située entre 20 et 40 W par g de Fe, de préférence entre 25 et 40 W par g de Fe, en particulier entre 30 et 40 W par g de Fe à une force de champ magnétique de 4 kA/m et à une fréquence d'un champ alternatif magnétique de 100 kHz.

22. Particules contenant du fer selon l'une des revendications 18 à 21, lesdites particules présentant un diamètre inférieur à 500 nm, de préférence de 1 à 100 nm, particulièrement de préférence de 15 à 30 nm.

23. Particules contenant du fer selon l'une des revendications 18 à 22, dans lesquelles l'enveloppe contenant du silicium est fonctionnalisée par des alkoxysilanes.

24. Particules contenant du fer selon la revendication 23, dans lesquelles les alkoxysilanes sont de préférence des trialkoxysilanes, qui
(i) portent un groupe fonctionnel lié par le biais d'une liaison Si-C, de préférence le (3-acryloxypropyl)-triméthoxysilane, le triéthoxysilylbutyraldéhyde, le 3-aminopropyltriéthoxysilane, le 3-isocyanatopropyltriéthoxysilane, ou
(ii) portent une chaîne latérale de polyéthylèneglycol liée par le biais d'une liaison Si-C, de longueur différente, de préférence le 2-[méthoxy(polyéthylènoxy)-propyl]triméthoxysilane.

25. Composition pharmaceutique comprenant des particules contenant du fer selon l'une des revendications 18 à 24.
